# EUROPEAN PATENT SPECIFICATION

(11) **EP 3 630 975 B1**
(45) Date of publication and mention of the grant of the patent: **19.11.2025**
(21) Application number: 18806333.3
(22) Date of filing: 24.05.2018
(51) Int. Cl.: A61K 47/54, C12N 15/10, C12N 15/90, C12N 9/22, C12N 15/11, C12N 15/63

(54) **ALTERED GUIDE RNAS FOR MODULATING CAS9 ACTIVITY AND METHODS OF USE**
VERÄNDERTE GUIDE-RNAS ZUR MODULIERUNG DER CAS9-AKTIVITÄT UND VERWENDUNGSVERFAHREN
ARN GUIDES MODIFIÉS POUR MODULER L'ACTIVITÉ CAS9 ET PROCÉDÉS D'UTILISATION

(30) Priority: 26.05.2017 US 201762511462 P
(43) Date of publication of application: 08.04.2020
(73) Proprietor: North Carolina State University, Raleigh, NC 27606 (US)
(72) Inventor: BARRANGOU, Rodolphe, Raleigh North Carolina 27695 (US); CRAWLEY, Alexandra Briner, Raleigh NorthCarolina 27695 (US)
(74) Representative: HGF
(86) International application number: PCT/US2018/034322
(87) International publication number: WO 2018/217981

(56) References cited:
- WO-A1-2017/027423
- WO-A1-2017/044776
- WO-A2-2015/112896
- WO-A9-2015/112896
- US-A1- 2014 186 919
- US-A1- 2016 186 213
- US-A1- 2016 186 213
- US-A1- 2017 002 339
- YING DANG ET AL: "Optimizing sgRNA structure to improve CRISPR-Cas9 knockout efficiency", GENOME BIOLOGY, vol. 16, no. 280, 1 January 2015 (2015-01-01), pages 1 - 10, XP055369116, DOI: 10.1186/s13059-015-0846-3
- BRINER A. ET AL.: "Guide RNA functional modules direct Cas9 activity and orthogonality", MOLECULAR CELL, vol. 56, no. 2, 2014, pages 333 - 339, XP055376599
- JINEK M. ET AL.: "A programmable dual-RNADNA endonuclease in adaptive bacterial immunity", SCIENCE, vol. 337, no. 6096, August 2012 (2012-08-01), pages 816 - 821, XP055549487

## Description

### STATEMENT REGARDING ELECTRONIC FILING OF A SEQUENCE LISTING

A Sequence Listing in ASCII text format, submitted under 37 C.F.R. § 1.821, entitled 5051-920WO_ST25.txt, 1,673,631 bytes in size, generated on May 22, 2018 and filed via EFS-Web, is provided in lieu of a paper copy. This Sequence Listing is hereby incorporated herein by reference into the specification for its disclosures.

### STATEMENT OF PRIORITY

This application claims the benefit, under 35 U.S.C. § 119 (e), of U.S. Provisional Application No. 62/511,462 filed on May 26, 2017.

### FIELD OF THE INVENTION

The invention relates to synthetic CRISPR-cas guides and methods of use thereof for site-specific cleavage and nicking, transcriptional control and DNA editing.

### BACKGROUND OF THE INVENTION

Clustered Regularly Interspaced Short Palindromic Repeats (CRISPR), in combination with associated sequences (*cas*) constitute the CRISPR-Cas system, which confers adaptive immunity in many bacteria. CRISPR-mediated immunization occurs through the uptake of DNA from invasive genetic elements such as plasmids and phages, as novel "spacers."

CRISPR-Cas systems consist of arrays of short DNA repeats interspaced by hypervariable sequences, flanked by *cas* genes, that provide adaptive immunity against invasive genetic elements such as phage and plasmids, through sequence-specific targeting and interference (Barrangou et al. 2007. Science. 315:1709-1712; Brouns et al. 2008. Science 321:960-4; Horvath and Barrangou. 2010. Science. 327:167-70; Marraffini and Sontheimer. 2008. Science. 322:1843-1845; Bhaya et al. 2011. Annu. Rev. Genet. 45:273-297; Terns and Terns. 2011. Curr. Opin. Microbiol. 14:321-327; Westra et al. 2012. Annu. Rev. Genet. 46:311-339; Barrangou R. 2013. RNA. 4:267-278). Typically, invasive DNA sequences are acquired as novel "spacers" (Barrangou et al. 2007. Science. 315:1709-1712), each paired with a CRISPR repeat and inserted as a novel repeat-spacer unit in the CRISPR locus. Subsequently, the repeat-spacer array is transcribed as a long pre-CRISPR RNA (pre-crRNA) (Brouns et al. 2008. Science 321:960-4), which is processed into small interfering CRISPR RNAs (crRNAs) that drive sequence-specific recognition. Specifically, crRNAs guide nucleases towards complementary targets for sequence-specific nucleic acid cleavage mediated by Cas endonucleases (Garneau et al. 2010. Nature. 468:67-71; Haurwitz et al. 2010. Science. 329:1355-1358; Sapranauskas et al. 2011. Nucleic Acid Res. 39:9275-9282; Jinek et al. 2012. Science. 337:816-821; Gasiunas et al. 2012. Proc. Natl. Acad. Sci. 109:E2579-E2586; Magadan et al. 2012. PLoS One. 7:e40913; Karvelis et al. 2013. RNA Biol. 10:841-851). These widespread systems occur in nearly half of bacteria (~46%) and the large majority of archaea (~90%). They are classified into six main CRISPR-Cas systems types (Makarova et al. 2011. Nature Rev. Microbiol. 9:467-477; Makarova et al. 2013. Nucleic Acid Res. 41:4360-4377; Makarova et al. 2015. Nature Rev. Microbiol. 13:722-736) based on the *cas* gene content, organization and variation in the biochemical processes that drive crRNA biogenesis, and Cas protein complexes that mediate target recognition and cleavage. In types I, III and IV, the specialized Cas endonucleases process the pre-crRNAs, which then assemble into a large multi-Cas protein complex capable of recognizing and cleaving nucleic acids complementary to the crRNA. A different process is involved in Type II CRISPR-Cas systems. Here, the pre-CRNAs are processed by a mechanism in which a trans-activating crRNA (tracrRNA) hybridizes to repeat regions of the crRNA. The hybridized crRNA-tracrRNA are cleaved by RNase III and following a second event that removes the 5' end of each spacer, mature crRNAs are produced that remain associated with the both the tracrRNA and Cas9. The mature complex then locates a target dsDNA sequence ('protospacer' sequence) that is complementary to the spacer sequence in the complex and cuts both strands. Target recognition and cleavage by the complex in the Type II system not only requires a sequence that is complementary between the spacer sequence on the crRNA-tracrRNA complex and the target 'protospacer' sequence but also requires a protospacer adjacent motif (PAM) sequence located at the 3' end of the protospacer sequence. The exact PAM sequence that is required can vary between different Type II systems.

The present disclosure provides modified guides and methods for making such guides for use in modulating the efficiency and specificity of synthetic Type II CRISPR-Cas systems in such uses as, for example, site-specific cleavage, site-specific nicking, transcriptional control and genome editing.
The state of the art is represented by the following publications:
US patent specification No. US2017002339A1 discloses methods and compositions for genome editing and DNA targeting of proteins and in particular, discloses method and compositions for sequences guiding Cas9 targeting.
International Patent publication No. WO2015112896A2 discloses methods and compositions for genome editing and DNA targeting of proteins and in particular, method and compositions for sequences guiding Cas9 targeting.
US patent specification No. US2016186213A1 discloses systems, methods, and compositions for manipulation of sequences and/or activities of target sequences. The publication discloses vectors and vector systems, some of which encode one or more components of a CRISPR complex, as well as methods for the design and use of such vectors. Also disclosed are methods of directing CRISPR complex formation in prokaryotic and eukaryotic cells to ensure enhanced specificity for target recognition and avoidance of toxicity.
YING DANG ET AL, "Optimizing sgRNA structure to improve CRISPR-Cas9 knockout efficiency", GENOME BIOLOGY, (2015-12-15), vol. 16, no. 280, doi:10.1186/s13059-015-0846-3, discloses investigation of sgRNA structure for increasing gene knockout efficiency in CRISPR-Cas9-based genome editing system.
International Patent publication No. WO2017027423A1 discloses engineered Type II CRISPR-Cas9-associated split-nexus polynucleotide (sn-casPNs) systems, including systems comprising three split- nexus polynucleotides (sn1-casPN/sn2-casPN/sn3-casPN) and systems comprising two split-nexus polynucleotides (sn1-casPN/sn2-casPN). The publication disclose that together with a Cas9 protein, the sn-casPNs facilitate site-specific modifications, including cleavage and mutagenesis, of a target polynucleotide in vitro and in vivo. Furthermore, the engineered Type II CRISPR-Cas9-associated split-nexus polynucleotide systems comprising sn-casPNs are disclosed as being useful in methods of regulating expression of a target nucleic acid.
BRINER A. et al., "Guide RNA functional modules direct Cas9 activity and orthogonality", Molecular cell, (2014-10-23), vol. 56, no. 2, pages 333 - 339, XP055376599 relates to the RNA-guided Cas9 endonuclease specifically targeting and cleaving DNA in a sequence-dependent manner and discloses that Cas9 activity is dependent on interactions with guide RNAs, and evolutionarily divergent Cas9 nucleases have been shown to work orthogonally. The authors note that the molecular basis of selective Cas9:guide-RNA interactions is poorly understood; and in this publication, the authors identify and characterize six conserved modules within native crRNA:tracrRNA duplexes and single guide RNAs (sgRNAs) that direct Cas9 endonuclease activity.
US Patent Specification No. US 2014/186919 A1 discloses compositions and methods related to components of a CRISPR complex particularly comprising a Cas ortholog enzyme.

### SUMMARY OF THE INVENTION

A first aspect of the invention provides a synthetic nucleic acid construct, comprising: (a) a crRNA comprising a 3' region and a 5' region, wherein the 3' region comprises at least about 10 consecutive nucleotides of a CRISPR repeat, and/or a functional fragment of the CRISPR repeat, and the 5' region comprises at least about 20 consecutive nucleotides of a spacer sequence located immediately upstream of the repeat; (b) a tracrRNA comprising a 5' and a 3' region, wherein at least a portion of the 5' region of the tracrRNA is complementary to the 3' region (i.e., CRISPR repeat) of the crRNA, wherein, when the 5' region of the tracrRNA that is complementary to the 3' region of the crRNA hybridizes to the 3' region of the crRNA, the synthetic nucleic acid construct forms secondary structures from 5' to 3' of: (i) a stem, the stem comprising a duplex between the 5' end of the tracrRNA and the repeat of the crRNA, and optionally, the stem further comprising a kink or a bulge; (ii) a nexus hairpin; (iii) at least one terminal hairpin; and (c) optionally, a linker that when present links the 3'end of the crRNA to the 5' end of the tracrRNA, wherein the construct comprises an insertion, substitution, and/or deletion of about one to about five nucleotides or base pairs in the nexus hairpin as compared to a wild type Type II crRNA:tracrRNA duplex; and/or an insertion, substitution and/or deletion of about one to about nine nucleotides and/or base pairs in the stem as compared to a wild type Type II crRNA:tracrRNA duplex.

A second aspect of the invention provides a protein-RNA complex, comprising: (a) a Cas9 polypeptide; and (b) a synthetic nucleic acid construct of the invention.

A third aspect of the invention provides a method of producing a modified Type II crRNA:tracrRNA construct, comprising: (a) inserting, substituting, and/or deleting about one to about five nucleotides or base pairs in the nexus hairpin of a Type II crRNA:tracrRNA; and/or (b) inserting, substituting, and/or deleting about one to about nine nucleotides and/or base pairs in a stem of the Type II crRNA:tracrRNA, wherein the crRNA comprises a 3' region and a 5' region, the 3' region comprising at least about 10 consecutive nucleotides of a CRISPR repeat, and/or a functional fragment of the repeat, and the 5' region comprises at least about 20 consecutive nucleotides of a spacer sequence located immediately upstream of the repeat, and the tracrRNA comprises a 5' and a 3' region, and at least a portion of the 5' region of the tracrRNA is complementary to the 3' region (i.e., CRISPR repeat) of the crRNA, further wherein, when the 5' region of the tracrRNA that is complementary to the 3' region of the crRNA hybridizes to the 3' region of the crRNA, the Type II crRNA:tracrRNA forms secondary structures from 5' to 3' of: (i) a stem, the stem comprising a duplex between the 5' end of the tracrRNA and the repeat of the crRNA, and optionally, the stem further comprising a kink or a bulge; (ii) a nexus hairpin; (iii) at least one terminal hairpin; and (c) optionally, a linker that when present links the 3'end of the crRNA to the 5' end of the tracrRNA (i.e., linking the 3' end and 5' end of the stem), thereby producing a modified Type II rRNA:tracrRNAs construct as compared to a wild type Type II crRNA:tracrRNA duplex.

A fourth aspect of the invention provides a method of modifying the activity of a Cas9 polypeptide, comprising complexing the Cas9 polypeptide with a synthetic nucleic acid construct of the invention, thereby modifying the activity of a Cas9 polypeptide as compared to a Cas9 polypeptide complexed with a wild type Type II crRNA:tracrRNA duplex.

A fifth aspect of the invention provides a method of controlling transcription of a target DNA, comprising: contacting the target DNA with a synthetic nucleic acid construct of the invention and/or an expression cassette and/or vector comprising the synthetic nucleic acid construct, in the presence of a Cas9 polypeptide, wherein the synthetic nucleic acid construct binds to the target DNA, thereby controlling the transcription of the target DNA.

A sixth aspect of the invention provides a method of controlling transcription of a target DNA, comprising: contacting the target DNA with a protein-RNA complex of the present invention, and/or an expression cassette and/or a vector comprising a synthetic nucleic acid construct and encoding a Cas9 polypeptide of the protein-RNA complex, wherein the protein RNA complex binds to the target DNA, thereby controlling the transcription of the target DNA.

A seventh aspect of the invention provides a method for site-specific cleavage of a target DNA, comprising: contacting the target DNA with a synthetic nucleic acid construct of the present invention, and/or an expression cassette and/or a vector comprising the synthetic nucleic acid construct, in the presence of a Cas9 polypeptide, thereby producing a site specific cleavage of the target DNA in a region defined by complementary binding of the spacer sequence of the crRNA of said synthetic nucleic acid construct to the target DNA.

An eighth aspect of the invention provides a method for site-specific cleavage of a target DNA, comprising: contacting the target DNA with a protein-RNA complex of the present invention, and/or an expression cassette and/or a vector comprising a synthetic nucleic acid construct and encoding a Cas9 polypeptide of the protein-RNA complex, thereby producing a site specific cleavage of the target DNA in a region defined by complementary binding of the spacer sequence of the crRNA of the protein-RNA complex to the target DNA.

A ninth aspect of the invention provides a method of editing a target DNA, comprising: contacting the target DNA with a synthetic nucleic acid construct of the present invention, and/or with an expression cassette and/or a vector comprising the synthetic nucleic acid construct, in the presence of a Cas9 polypeptide, wherein the synthetic nucleic acid construct binds to the target DNA, thereby editing the target DNA.

A tenth aspect of the invention provides a method of editing a target DNA, comprising: contacting the target DNA with a protein-RNA complex of the present invention, and/or an expression cassette and/or a vector comprising a synthetic nucleic acid construct and encoding a Cas9 polypeptide of the protein-RNA complex, wherein the protein-RNA complex binds to the target DNA, thereby editing the target DNA.

An eleventh aspect of the invention provides a method for site-specific nicking of a (+) strand of a double stranded target DNA, comprising: contacting the double stranded target DNA with a protein-RNA complex of the present invention, and/or an expression cassette and/or a vector comprising a synthetic nucleic acid construct and encoding a Cas9 polypeptide of the protein-RNA complex, wherein the Cas9 polypeptide, comprises a point mutation in an RuvC active site motif, and the Cas9 polypeptide cleaves the (+) strand of the double stranded DNA, thereby producing a site-specific nick in said double stranded target DNA.

A twelfth aspect of the invention provides a method for site-specific nicking of a (-) strand of a double stranded target DNA, comprising: contacting the double stranded target DNA with a protein-RNA complex of the present invention, and/or an expression cassette and/or a vector comprising a synthetic nucleic acid construct and encoding a Cas9 polypeptide of the protein-RNA complex, wherein the Cas9 polypeptide, comprises a point mutation in an HNH active site motif, and the Cas9 polypeptide cleaves the (-) strand of the double stranded DNA, thereby producing a site-specific nick in said double stranded target DNA.

The invention further provides expression cassettes, vectors and cells comprising the synthetic nucleic acids constructs of this invention.

These and other aspects of the invention are set forth in more detail in the description of the invention below.

### BRIEF DESCRIPTION OF THE SEQUENCES IN THE SEQUENCE LISTING

**SEQ ID NOs:1-98** are example nucleotide sequences comprising wild type crRNA repeat sequences useful with this invention.
**SEQ ID NOs:99-193** are example wild type tracrRNA nucleotide sequences useful with this invention.
**SEQ ID NOs:194-293** are example Cas9 polypeptide sequences useful with the present invention.
**SEQ ID NOs:294-388** are the example Cas9 nucleotide sequences useful with this invention and encoding the respective Cas9 polypeptide sequences, **SEQ ID NOs:194-293.**

### BRIEF DESCRIPTION OF THE DRAWINGS

**Fig. 1** show the process for modifying and selecting mutant single guides for *Lactobacillus gasseri.* **Panel A** shows the wild type *L. gasseri* duplex and an example of a synthetic mutant guide of the invention. **Panel B** shows cloning of a guide into a transformation vector. **Fig. 1****, panels C-E** show the process for distinguish the functionality of the different mutant guides following of the transformation of the mutant guides into *L*. *gasseri.*

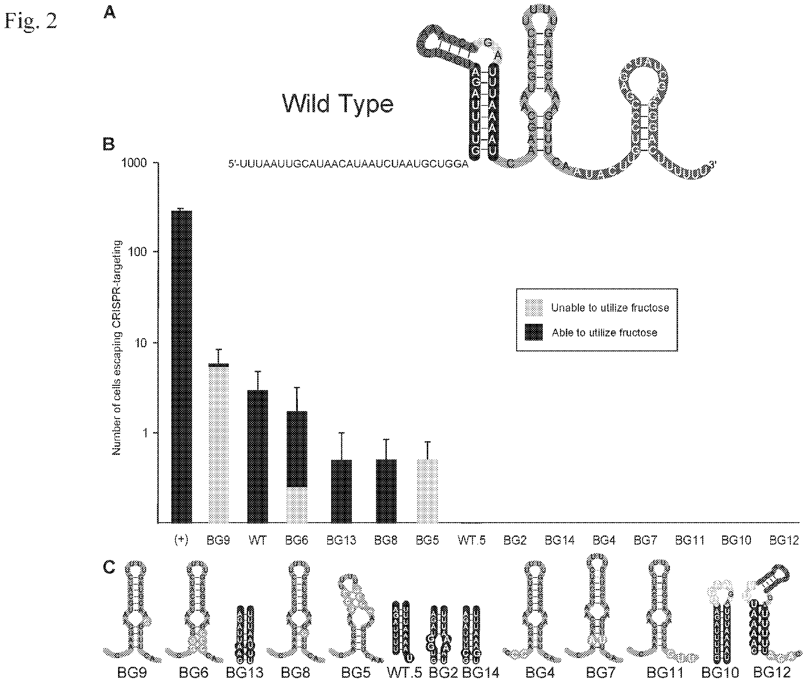

**Fig. 2** show the wild type single guide *Lactobacillus gasseri* (Lga) Type II crRNA:tracrRNA duplex and mutant single guides based on the Lga Type II duplex. **Panel A** shows the wild type single guide RNA sequence (**SEQ ID NO:403**) and structure. The 5 modules of guide RNAs are provided left to right as follows: stem (lower stem (black), bulge (light grey), upper stem (dark grey), nexus (medium grey), terminal hairpin(s) (dark grey). **Panel B** provides a graph of the data showing recovered transformants from sgRNA delivery. The ability of recovered cells to utilize fructose is shown by the black and grey stacked bars. Grey demonstrates cells that were unable to utilize fructose, while black demonstrates native ability to utilize fructose. **Panel C** shows the mutations made to each guide (**SEQ ID NOs:404-412**). Only the module of the guide that was mutated is shown. The specific nucleotides that were mutated are in white surrounded by a circle and/or are in larger font than the non-mutated nucleotides.
**Fig. 3** shows the full structures of wild type (**SEQ ID NO:403**) and example mutant single guides (**SEQ ID NOs:413-426**) discussed in **Fig. 2****, Panels A-C.** Mutations are shown as bold and larger font than the non-mutated nucleotides.

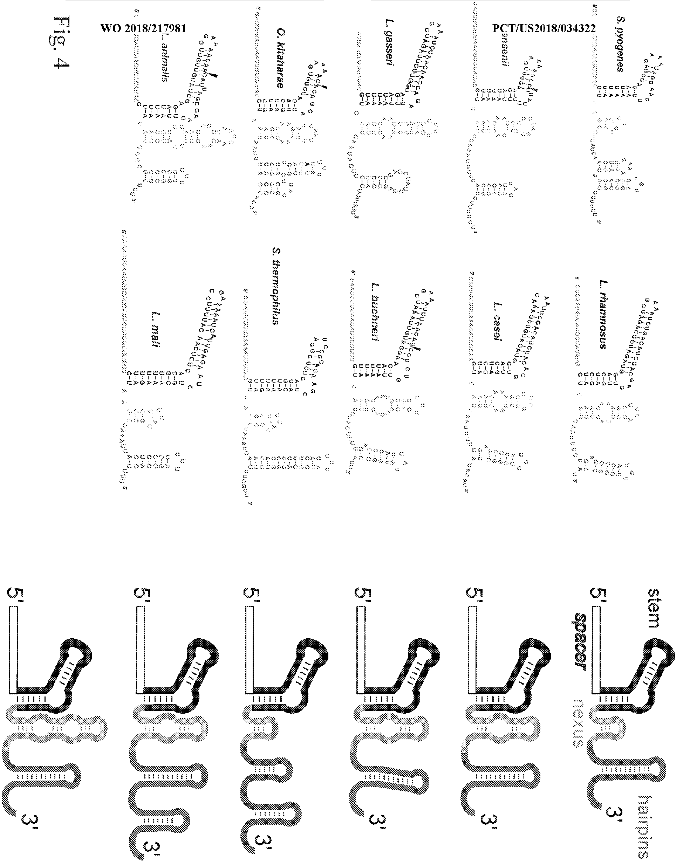

**Fig. 4** shows example WT single guides (**SEQ ID NOs:427-436**) for various bacteria that may be used as templates for modifying single guides as described herein: *Streptococcus pyrogenes, Lactobacillus rhamnosus, Lactobacillus jensenii, Lactobacillus casei*, *Lactobacillus gasseri, Lactobacillus buchneri, Oenococcus kitaharae, Streptococcus thermophilus CRISPR1, Lactobacillus animalis* and *Lactobacillus mali.* The rights panel shows the generic structures of these guides without nucleotides. The spacer, stem, nexus and hairpin regions are distinguished in each of the structures by the different shades of grey or black.
**Fig. 5** shows CRISPR repeats (**SEQ ID NOs:437-458**) sequence alignment. For each cluster, CRISPR repeat sequence alignments are shown, with conserved and consensus nucleotides specified at the bottom of each family, with Sth3 (top), Sth1 (middle) and Lb (bottom) families.
**Fig. 6** shows tracrRNA and repeat sequence alignments for *Streptococcus* species/strains.
**Fig. 7** shows tracrRNA and repeat sequence alignments for *Streptococcus* species/strains.
**Fig. 8** shows tracrRNA and repeat sequence alignments for *Lactobacillus* species/strains.
**Fig. 9** shows tracrRNA and repeat sequence alignments for *Lactobacillus* species/strains.
**Fig. 10** provides example repeat sequences and tracrRNA sequence from various organisms.

### DETAILED DESCRIPTION OF THE INVENTION

The present invention now will be described hereinafter with reference to the accompanying drawings and examples, in which embodiments of the invention are shown. This description is not intended to be a detailed catalog of all the different ways in which the invention may be implemented, or all the features that may be added to the instant invention. For example, features illustrated with respect to one embodiment may be incorporated into other embodiments, and features illustrated with respect to a particular embodiment may be deleted from that embodiment. Thus, the invention contemplates that in some embodiments of the invention, any feature or combination of features set forth herein can be excluded or omitted. In addition, numerous variations and additions to the various embodiments suggested herein will be apparent to those skilled in the art in light of the instant disclosure, which do not depart from the instant invention. Hence, the following descriptions are intended to illustrate some particular embodiments of the invention, and not to exhaustively specify all permutations, combinations and variations thereof.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

All publications, patent applications, patents and other references cited herein are incorporated by reference in their entireties for the teachings relevant to the sentence and/or paragraph in which the reference is presented.

Unless the context indicates otherwise, it is specifically intended that the various features and embodiments of the invention described herein can be used in any combination. Moreover, the present invention also contemplates that in some embodiments of the invention, any feature or combination of features set forth herein can be excluded or omitted. To illustrate, if the specification states that a composition comprises components A, B and C, it is specifically intended that any of A, B or C, or a combination thereof, can be omitted and disclaimed singularly or in any combination.

As used in the description of the invention and the appended claims, the singular forms "a," "an" and "the" are intended to include the plural forms as well, unless the context clearly indicates otherwise.

Also as used herein, "and/or" refers to and encompasses any and all possible combinations of one or more of the associated listed items, as well as the lack of combinations when interpreted in the alternative ("or").

The term "about," as used herein when referring to a measurable value such as a dosage or time period and the like, is meant to encompass variations of ± 20%, ± 10%, ± 5%, ± 1%, ± 0.5%, or even ± 0.1% of the specified amount.

As used herein, phrases such as "between X and Y" and "between about X and Y" should be interpreted to include X and Y. As used herein, phrases such as "between about X and Y" mean "between about X and about Y" and phrases such as "from about X to Y" mean "from about X to about Y."

The term "comprise," "comprises" and "comprising" as used herein, specify the presence of the stated features, integers, steps, operations, elements, and/or components, but do not preclude the presence or addition of one or more other features, integers, steps, operations, elements, components, and/or groups thereof.

As used herein, the transitional phrase "consisting essentially of" means that the scope of a claim is to be interpreted to encompass the specified materials or steps recited in the claim and those that do not materially affect the basic and novel characteristic(s) of the claimed invention. Thus, the term "consisting essentially of" when used in a claim of this invention is not intended to be interpreted to be equivalent to "comprising."

As used herein, the terms "increase," "increasing," "increased," "enhance," "enhanced," "enhancing," and "enhancement" (and grammatical variations thereof) describe an elevation of at least about 25%, 50%, 75%, 100%, 150%, 200%, 300%, 400%, 500% or more as compared to a control.

As used herein, the terms "reduce," "reduced," "reducing," "reduction," "diminish," "suppress," and "decrease" (and grammatical variations thereof), describe, for example, a decrease of at least about 5%, 10%, 15%, 20%, 25%, 35%, 50%, 75%, 80%, 85%, 90%, 95%, 97% or more, as compared to a control. In particular embodiments, the reduction results in no or essentially no (*i.e.,* an insignificant amount, *e.g.*, less than about 10% or even 5%) detectable activity or amount.

A "heterologous" or a "recombinant" nucleotide sequence is a nucleotide sequence not naturally associated with a host cell into which it is introduced, including non- naturally occurring multiple copies of a naturally occurring nucleotide sequence.

A "native" or "wild type" nucleic acid, nucleotide sequence, polypeptide or amino acid sequence refers to a naturally occurring or endogenous nucleic acid, nucleotide sequence, polypeptide or amino acid sequence. Thus, for example, a "wild type mRNA" is an mRNA that is naturally occurring in or endogenous to the organism. A "homologous" nucleic acid sequence is a nucleotide sequence naturally associated with a host cell into which it is introduced.

As used herein, the terms "nucleic acid," "nucleic acid molecule," "nucleotide sequence" and "polynucleotide" refer to RNA or DNA that is linear or branched, single or double stranded, or a hybrid thereof. The term also encompasses RNA/DNA hybrids. When dsRNA is produced synthetically, less common bases, such as inosine, 5-methylcytosine, 6-methyladenine, hypoxanthine and others can also be used for antisense, dsRNA, and ribozyme pairing. For example, polynucleotides that contain C-5 propyne analogues of uridine and cytidine have been shown to bind RNA with high affinity and to be potent antisense inhibitors of gene expression. Other modifications, such as modification to the phosphodiester backbone, or the 2'-hydroxy in the ribose sugar group of the RNA can also be made.

As used herein, the term "nucleotide sequence" refers to a heteropolymer of nucleotides or the sequence of these nucleotides from the 5' to 3' end of a nucleic acid molecule and includes DNA or RNA molecules, including cDNA, a DNA fragment or portion, genomic DNA, synthetic (*e.g*., chemically synthesized) DNA, plasmid DNA, mRNA, and anti-sense RNA, any of which can be single stranded or double stranded. The terms "nucleotide sequence" "nucleic acid," "nucleic acid molecule," "oligonucleotide" and "polynucleotide" are also used interchangeably herein to refer to a heteropolymer of nucleotides. Nucleic acid molecules and/or nucleotide sequences provided herein are presented herein in the 5' to 3' direction, from left to right and are represented using the standard code for representing the nucleotide characters as set forth in the U.S. sequence rules, 37 CFR §§1.821 - 1.825 and the World Intellectual Property Organization (WIPO) Standard ST.25. A "5' region" as used herein can mean the region of a polynucleotide that is nearest the 5' end. Thus, for example, an element in the 5' region of a polynucleotide can be located anywhere from the first nucleotide located at the 5' end of the polynucleotide to the nucleotide located halfway through the polynucleotide. A "3' region" as used herein can mean the region of a polynucleotide that is nearest the 3' end. Thus, for example, an element in the 3' region of a polynucleotide can be located anywhere from the first nucleotide located at the 3' end of the polynucleotide to the nucleotide located halfway through the polynucleotide.

As used herein, the term "gene" refers to a nucleic acid molecule capable of being used to produce mRNA, antisense RNA, miRNA, anti-microRNA antisense oligodeoxyribonucleotide (AMO) and the like. Genes may or may not be capable of being used to produce a functional protein or gene product. Genes can include both coding and non-coding regions (e.g., introns, regulatory elements, promoters, enhancers, termination sequences and/or 5' and 3' untranslated regions). A gene may be "isolated" by which is meant a nucleic acid that is substantially or essentially free from components normally found in association with the nucleic acid in its natural state. Such components include other cellular material, culture medium from recombinant production, and/or various chemicals used in chemically synthesizing the nucleic acid.

The terms "complementary" or "complementarity," as used herein, refer to the natural binding of polynucleotides under permissive salt and temperature conditions by base-pairing. For example, the sequence "A-G-T" (5' to 3') binds to the complementary sequence "T-C-A" (3' to 5'). Complementarity between two single-stranded molecules may be "partial," in which only some of the nucleotides bind, or it may be complete when total complementarity exists between the single stranded molecules. The degree of complementarity between nucleic acid strands has significant effects on the efficiency and strength of hybridization between nucleic acid strands.

"Complement" as used herein can mean 100% complementarity or identity with the comparator nucleotide sequence or it can mean less than 100% complementarity (e.g., about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, and the like, complementarity).

As used herein, "a corresponding wild type guide" refers to a wild type guide that corresponds to a synthetic guide of the present invention (i.e., the synthetic nucleic acid construct). That is, a synthetic guide is based on a corresponding wild type guide but the synthetic guide differs from the corresponding wild type guide by the mutations that are inserted in the stem and nexus hairpin regions of the synthetic guide as described herein. Further, a synthetic guide also corresponds to native crRNA and tracrRNA sequences linked artificially at the 3' end of the crRNA and the 5' end of the tracrRNA without any modifications to the stem or nexus regions of the native crRNA and tracrRNA sequences.

A "portion" or "fragment" of a nucleotide sequence of the invention will be understood to mean a nucleotide sequence of reduced length relative (e.g., reduced by 1, 2, 3, 4, 5, 6, 7, 8, 9, 10, 11, 12, 13, 14, 15, 16, 17, 18, 19, 20 or more nucleotides) to a reference nucleic acid or nucleotide sequence and comprising, consisting essentially of and/or consisting of a nucleotide sequence of contiguous nucleotides identical or almost identical (e.g., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identical) to the reference nucleic acid or nucleotide sequence. Such a nucleic acid fragment or portion according to the invention may be, where appropriate, included in a larger polynucleotide of which it is a constituent.

Different nucleic acids or proteins having homology are referred to herein as "homologues." The term homologue includes homologous sequences from the same and other species and orthologous sequences from the same and other species. "Homology" refers to the level of similarity between two or more nucleic acid and/or amino acid sequences in terms of percent of positional identity (*i.e.,* sequence similarity or identity). Homology also refers to the concept of similar functional properties among different nucleic acids or proteins. Thus, the compositions and methods of the invention further comprise homologues to the nucleotide sequences and polypeptide sequences of this invention. "Orthologous," as used herein, refers to homologous nucleotide sequences and/ or amino acid sequences in different species that arose from a common ancestral gene during speciation. A homologue of a nucleotide sequence of this invention has a substantial sequence identity (e.g., at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, and/or 100%) to said nucleotide sequence of the invention.

As used herein "sequence identity" refers to the extent to which two optimally aligned polynucleotide or peptide sequences are invariant throughout a window of alignment of components, e.g., nucleotides or amino acids. "Identity" can be readily calculated by known methods including, but not limited to, those described in: Computational Molecular Biology (Lesk, A. M., ed.) Oxford University Press, New York (1988); Biocomputing: Informatics and Genome Projects (Smith, D. W., ed.) Academic Press, New York (1993); Computer Analysis of Sequence Data, Part I (Griffin, A. M., and Griffin, H. G., eds.) Humana Press, New Jersey (1994); Sequence Analysis in Molecular Biology (von Heinje, G., ed.) Academic Press (1987); and Sequence Analysis Primer (Gribskov, M. and Devereux, J., eds.) Stockton Press, New York (1991).

As used herein, the term "percent sequence identity" or "percent identity" refers to the percentage of identical nucleotides in a linear polynucleotide sequence of a reference ("query") polynucleotide molecule (or its complementary strand) as compared to a test ("subject") polynucleotide molecule (or its complementary strand) when the two sequences are optimally aligned. In some embodiments, "percent identity" can refer to the percentage of identical amino acids in an amino acid sequence.

As used herein, the phrase "substantially identical," or "substantial identity" in the context of two nucleic acid molecules, nucleotide sequences or protein sequences, refers to two or more sequences or subsequences that have at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, and/or 100% nucleotide or amino acid residue identity, when compared and aligned for maximum correspondence, as measured using one of the following sequence comparison algorithms or by visual inspection. In some embodiments of the invention, the substantial identity exists over a region of consecutive nucleotides of a nucleotide sequence of the invention that is about 16 nucleotides to about 30 nucleotides, about 18 nucleotides to about 25 nucleotides, about 30 nucleotides to about 40 nucleotides, about 50 nucleotides to about 60 nucleotides, about 70 nucleotides to about 80 nucleotides, about 90 nucleotides to about 100 nucleotides, or more nucleotides in length, and any range therein, up to the full length of the sequence. In some embodiments, the nucleotide sequences can be substantially identical over at least about 22 nucleotides. In some embodiments, the nucleotide sequences can be substantially identical over at least about 20 nucleotides. In some embodiments, a substantially identical nucleotide or protein sequence performs substantially the same function as the nucleotide or protein sequence to which it is substantially identical.

For sequence comparison, typically one sequence acts as a reference sequence to which test sequences are compared. When using a sequence comparison algorithm, test and reference sequences are entered into a computer, subsequence coordinates are designated if necessary, and sequence algorithm program parameters are designated. The sequence comparison algorithm then calculates the percent sequence identity for the test sequence(s) relative to the reference sequence, based on the designated program parameters.

Optimal alignment of sequences for aligning a comparison window are well known to those skilled in the art and may be conducted by tools such as the local homology algorithm of Smith and Waterman, the homology alignment algorithm of Needleman and Wunsch, the search for similarity method of Pearson and Lipman, and optionally by computerized implementations of these algorithms such as GAP, BESTFIT, FASTA, and TFASTA available as part of the GCG^{®} Wisconsin Package^{®} (Accelrys Inc., San Diego, CA). An "identity fraction" for aligned segments of a test sequence and a reference sequence is the number of identical components which are shared by the two aligned sequences divided by the total number of components in the reference sequence segment, *i.e.*, the entire reference sequence or a smaller defined part of the reference sequence. Percent sequence identity is represented as the identity fraction multiplied by 100. The comparison of one or more polynucleotide sequences may be to a full-length polynucleotide sequence or a portion thereof, or to a longer polynucleotide sequence. For purposes of this invention "percent identity" may also be determined using BLASTX version 2.0 for translated nucleotide sequences and BLASTN version 2.0 for polynucleotide sequences.

Two nucleotide sequences may also be considered substantially complementary when the two sequences hybridize to each other under stringent conditions. In some representative embodiments, two nucleotide sequences considered to be substantially complementary hybridize to each other under highly stringent conditions.

"Stringent hybridization conditions" and "stringent hybridization wash conditions" in the context of nucleic acid hybridization experiments such as Southern and Northern hybridizations are sequence dependent, and are different under different environmental parameters. An extensive guide to the hybridization of nucleic acids is found in Tijssen Laboratory Techniques in Biochemistry and Molecular Biology-Hybridization with Nucleic Acid Probes part I chapter 2 "Overview of principles of hybridization and the strategy of nucleic acid probe assays" Elsevier, New York (1993). Generally, highly stringent hybridization and wash conditions are selected to be about 5°C lower than the thermal melting point (Tₘ) for the specific sequence at a defined ionic strength and pH.

The Tₘ is the temperature (under defined ionic strength and pH) at which 50% of the target sequence hybridizes to a perfectly matched probe. Very stringent conditions are selected to be equal to the Tₘ for a particular probe. An example of stringent hybridization conditions for hybridization of complementary nucleotide sequences which have more than 100 complementary residues on a filter in a Southern or northern blot is 50% formamide with 1 mg of heparin at 42°C, with the hybridization being carried out overnight. An example of highly stringent wash conditions is 0.1 5M NaCl at 72°C for about 15 minutes. An example of stringent wash conditions is a 0.2x SSC wash at 65°C for 15 minutes (*see,* Sambrook, *infra*, for a description of SSC buffer). Often, a high stringency wash is preceded by a low stringency wash to remove background probe signal. An example of a medium stringency wash for a duplex of, e.g., more than 100 nucleotides, is 1x SSC at 45°C for 15 minutes. An example of a low stringency wash for a duplex of, *e.g.,* more than 100 nucleotides, is 4-6x SSC at 40°C for 15 minutes. For short probes (e.g., about 10 to 50 nucleotides), stringent conditions typically involve salt concentrations of less than about 1.0 M Na ion, typically about 0.01 to 1.0 M Na ion concentration (or other salts) at pH 7.0 to 8.3, and the temperature is typically at least about 30°C. Stringent conditions can also be achieved with the addition of destabilizing agents such as formamide. In general, a signal to noise ratio of 2x (or higher) than that observed for an unrelated probe in the particular hybridization assay indicates detection of a specific hybridization. Nucleotide sequences that do not hybridize to each other under stringent conditions are still substantially identical if the proteins that they encode are substantially identical. This can occur, for example, when a copy of a nucleotide sequence is created using the maximum codon degeneracy permitted by the genetic code.

The following are examples of sets of hybridization/wash conditions that may be used to clone homologous nucleotide sequences that are substantially identical to reference nucleotide sequences of the invention. In one embodiment, a reference nucleotide sequence hybridizes to the "test" nucleotide sequence in 7% sodium dodecyl sulfate (SDS), 0.5 M NaPO₄, 1 mM EDTA at 50°C with washing in 2X SSC, 0.1% SDS at 50°C. In another embodiment, the reference nucleotide sequence hybridizes to the "test" nucleotide sequence in 7% sodium dodecyl sulfate (SDS), 0.5 M NaPO₄, 1 mM EDTA at 50°C with washing in 1X SSC, 0.1% SDS at 50°C or in 7% sodium dodecyl sulfate (SDS), 0.5 M NaPO₄, 1 mM EDTA at 50°C with washing in 0.5X SSC, 0.1% SDS at 50°C. In still further embodiments, the reference nucleotide sequence hybridizes to the "test" nucleotide sequence in 7% sodium dodecyl sulfate (SDS), 0.5 M NaPO₄, 1 mM EDTA at 50°C with washing in 0.1X SSC, 0.1% SDS at 50°C, or in 7% sodium dodecyl sulfate (SDS), 0.5 M NaPO₄, 1 mM EDTA at 50°C with washing in 0.1X SSC, 0.1% SDS at 65°C.

Any nucleotide sequence and/or recombinant nucleic acid molecule of this invention can be codon optimized for expression in any organism of interest. Codon optimization is well known in the art and involves modification of a nucleotide sequence for codon usage bias using species specific codon usage tables. The codon usage tables are generated based on a sequence analysis of the most highly expressed genes for the organism/species of interest. When the nucleotide sequences are to be expressed in the nucleus, the codon usage tables are generated based on a sequence analysis of highly expressed nuclear genes for the species of interest. The modifications of the nucleotide sequences are determined by comparing the species specific codon usage table with the codons present in the native polynucleotide sequences. As is understood in the art, codon optimization of a nucleotide sequence results in a nucleotide sequence having less than 100% identity (e.g., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, and the like) to the native nucleotide sequence but which still encodes a polypeptide having the same function as that encoded by the original, native nucleotide sequence. Thus, in some embodiments of the invention, the synthetic nucleic acid constructs, expression cassettes, and/or vectors of the invention may be codon optimized for expression in the particular plant species of interest. In some embodiments, the codon optimized synthetic nucleic acid constructs, expression cassettes, and/or vectors have about 70% to about 99% identity to the synthetic nucleic acid constructs, expression cassettes, and/or vectors of the invention.

In any of the embodiments described herein, a synthetic nucleic acid construct of the invention may be operatively associated with a variety of promoters and other regulatory elements for expression in an organism of interest and/or a cell of an organism of interest. Thus, in some embodiments, a synthetic nucleic acid construct of this invention may further comprise one or more promoters operably linked to one or more nucleotide sequences.

By "operably linked" or "operably associated" as used herein, it is meant that the indicated elements are functionally related to each other, and are also generally physically related. Thus, the term "operably linked" or "operably associated" as used herein, refers to nucleotide sequences on a single nucleic acid molecule that are functionally associated. Thus, a first nucleotide sequence that is operably linked to a second nucleotide sequence means a situation when the first nucleotide sequence is placed in a functional relationship with the second nucleotide sequence. For instance, a promoter is operably associated with a nucleotide sequence if the promoter effects the transcription or expression of said nucleotide sequence. Those skilled in the art will appreciate that the control sequences (e.g., promoter) need not be contiguous with the nucleotide sequence to which it is operably associated, as long as the control sequences function to direct the expression thereof. Thus, for example, intervening untranslated, yet transcribed, sequences can be present between a promoter and a nucleotide sequence, and the promoter can still be considered "operably linked" to the nucleotide sequence.

A "promoter" is a nucleotide sequence that controls or regulates the transcription of a nucleotide sequence (*i.e.,* a coding sequence) that is operably associated with the promoter. The coding sequence may encode a polypeptide and/or a functional RNA. Typically, a "promoter" refers to a nucleotide sequence that contains a binding site for RNA polymerase II and directs the initiation of transcription. In general, promoters are found 5', or upstream, relative to the start of the coding region of the corresponding coding sequence. The promoter region may comprise other elements that act as regulators of gene expression. These include a TATA box consensus sequence, and often a CAAT box consensus sequence (Breathnach and Chambon, (1981) Annu. Rev. Biochem. 50:349). In plants, the CAAT box may be substituted by the AGGA box (Messing et al., (1983) in Genetic Engineering of Plants, T. Kosuge, C. Meredith and A. Hollaender (eds.), Plenum Press, pp. 211-227).

Promoters can include, for example, constitutive, inducible, temporally regulated, developmentally regulated, chemically regulated, tissue-preferred and/or tissue-specific promoters for use in the preparation of recombinant nucleic acid molecules, i.e., "synthetic nucleic acid constructs" or "protein-RNA complex." These various types of promoters are known in the art.

The choice of promoter may vary depending on the temporal and spatial requirements for expression, and also may vary based on the host cell to be transformed. Promoters for many different organisms are well known in the art. Based on the extensive knowledge present in the art, the appropriate promoter can be selected for the particular host organism of interest. Thus, for example, much is known about promoters upstream of highly constitutively expressed genes in model organisms and such knowledge can be readily accessed and implemented in other systems as appropriate.

In some embodiments, a synthetic nucleic acid construct of the invention and/or a protein-RNA complex of the invention can be an "expression cassette" or can be comprised within an expression cassette. As used herein, "expression cassette" means a recombinant nucleic acid molecule comprising, for example, a synthetic nucleic acid construct of the invention (e.g., crRNA, tracrRNA), wherein the synthetic nucleic acid construct of the invention is operably associated with at least a control sequence (e.g., a promoter). Thus, some embodiments of the invention provide expression cassettes designed to express, for example, a synthetic nucleic acid construct of the invention.

An expression cassette comprising a nucleotide sequence of interest (e.g., crRNA, tracrRNA) may be chimeric, meaning that at least one of its components is heterologous with respect to at least one of its other components. An expression cassette may also be one that is naturally occurring but has been obtained in a recombinant form useful for heterologous expression.

An expression cassette also can optionally include a transcriptional and/or translational termination region (*i.e.,* termination region) that is functional in the selected host cell. A variety of transcriptional terminators are available for use in expression cassettes and are responsible for the termination of transcription beyond the heterologous nucleotide sequence of interest and correct mRNA polyadenylation. The termination region may be native to the transcriptional initiation region, may be native to the operably linked nucleotide sequence of interest, may be native to the host cell, or may be from another source (*i.e.*, foreign or heterologous to the promoter, to the nucleotide sequence of interest, to the host, or any combination thereof).

An expression cassette of the invention also can include a nucleotide sequence for a selectable marker, which can be used to select a transformed host cell. As used herein, "selectable marker" means a nucleotide sequence that when expressed imparts a distinct phenotype to the host cell expressing the marker and thus allows such transformed cells to be distinguished from those that do not have the marker. Such a nucleotide sequence may encode either a selectable or screenable marker, depending on whether the marker confers a trait that can be selected for by chemical means, such as by using a selective agent (*e.g.*, an antibiotic and the like), or on whether the marker is simply a trait that one can identify through observation or testing, such as by screening (*e.g*., fluorescence). Of course, many examples of suitable selectable markers are known in the art and can be used in the expression cassettes described herein.

In addition to expression cassettes, the nucleic acid molecules and nucleotide sequences described herein can be used in connection with vectors. The term "vector" refers to a composition for transferring, delivering or introducing a nucleic acid (or nucleic acids) into a cell. A vector comprises a nucleic acid molecule comprising the nucleotide sequence(s) to be transferred, delivered or introduced. Vectors for use in transformation of host organisms are well known in the art. Non-limiting examples of general classes of vectors include but are not limited to a viral vector, a plasmid vector, a phage vector, a phagemid vector, a cosmid vector, a fosmid vector, a bacteriophage, an artificial chromosome, or an Agrobacterium binary vector in double or single stranded linear or circular form which may or may not be self transmissible or mobilizable. A vector as defined herein can transform prokaryotic or eukaryotic host either by integration into the cellular genome or exist extrachromosomally (e.g. autonomous replicating plasmid with an origin of replication). Additionally included are shuttle vectors by which is meant a DNA vehicle capable, naturally or by design, of replication in two different host organisms, which may be selected from actinomycetes and related species, bacteria and eukaryotic (e.g. higher plant, mammalian, yeast or fungal cells). In some embodiments, the nucleic acid in the vector is under the control of, and operably linked to, an appropriate promoter or other regulatory elements for transcription in a host cell. The vector may be a bi-functional expression vector which functions in multiple hosts. In the case of genomic DNA, this may contain its own promoter or other regulatory elements and in the case of cDNA this may be under the control of an appropriate promoter or other regulatory elements for expression in the host cell. Accordingly, a synthetic nucleic acid construct of this invention, a protein-RNA complex of the invention, and/or expression cassettes can be comprised in vectors as described herein and as known in the art.

As used herein, "contact", contacting", "contacted," and grammatical variations thereof, refers to placing the components of a desired reaction together under conditions suitable for carrying out the desired reaction (e.g., transcriptional control, genome editing, nicking, cleavage, and/or amplifying nucleic acids).

"Introducing," "introduce," "introduced" (and grammatical variations thereof) in the context of a polynucleotide of interest means presenting a nucleotide sequence of interest (e.g., a synthetic nucleic acid construct, a protein-RNA complex, a crRNA, tracrRNA, and/or Cas9 polynucleotide) to the host organism or cell of said organism (e.g., host cell) in such a manner that the nucleotide sequence gains access to the interior of a cell. Where more than one nucleotide sequence is to be introduced these nucleotide sequences can be assembled as part of a single polynucleotide or nucleic acid construct, or as separate polynucleotide or nucleic acid constructs, and can be located on the same or different expression constructs or transformation vectors. Accordingly, these polynucleotides can be introduced into a host cell in a single transformation event, in separate transformation events, or, for example, they can be incorporated into an organism by conventional breeding protocols.

The term "transformation" as used herein refers to the introduction of a heterologous nucleic acid into a cell. Transformation of a cell may be stable or transient. Thus, in some embodiments, a host cell or host organism is stably transformed with a nucleic acid molecule of the invention. In other embodiments, a host cell or host organism is transiently transformed with a recombinant nucleic acid molecule of the invention.

"Transient transformation" in the context of a polynucleotide means that a polynucleotide is introduced into the cell and does not integrate into the genome of the cell.

By "stably introducing" or "stably introduced" in the context of a polynucleotide introduced into a cell is intended that the introduced polynucleotide is stably incorporated into the genome of the cell, and thus the cell is stably transformed with the polynucleotide.

"Stable transformation" or "stably transformed" as used herein means that a nucleic acid molecule is introduced into a cell and integrates into the genome of the cell. As such, the integrated nucleic acid molecule is capable of being inherited by the progeny thereof, more particularly, by the progeny of multiple successive generations. "Genome" as used herein also includes the nuclear and the plastid genome, and therefore includes integration of the nucleic acid into, for example, the chloroplast or mitochondrial genome. Stable transformation as used herein can also refer to a transgene that is maintained extrachromasomally, for example, as a minichromosome or a plasmid.

Transient transformation may be detected by, for example, an enzyme-linked immunosorbent assay (ELISA) or Western blot, which can detect the presence of a peptide or polypeptide encoded by one or more transgene introduced into an organism. Stable transformation of a cell can be detected by, for example, a Southern blot hybridization assay of genomic DNA of the cell with nucleic acid sequences which specifically hybridize with a nucleotide sequence of a transgene introduced into an organism (e.g., a plant). Stable transformation of a cell can be detected by, for example, a Northern blot hybridization assay of RNA of the cell with nucleic acid sequences which specifically hybridize with a nucleotide sequence of a transgene introduced into a host organism. Stable transformation of a cell can also be detected by, *e.g.*, a polymerase chain reaction (PCR) or other amplification reactions as are well known in the art, employing specific primer sequences that hybridize with target sequence(s) of a transgene, resulting in amplification of the transgene sequence, which can be detected according to standard methods Transformation can also be detected by direct sequencing and/or hybridization protocols well known in the art.

Accordingly, in some embodiments, the nucleotide sequences, constructs, expression cassettes can be expressed transiently and/or they can be stably incorporated into the genome of the host organism. Thus, in some embodiments, a Cas9 polypeptide may be introduced into a cell with a RNA guide and as such no DNA is introduced or maintained in the cell.

A synthetic nucleic acid construct/polynucleotide of the invention can be introduced into a cell by any method known to those of skill in the art. In some embodiments of the invention, transformation of a cell comprises nuclear transformation. In other embodiments, transformation of a cell comprises plastid transformation (e.g., chloroplast transformation). In still further embodiments, the recombinant nucleic acid molecule/polynucleotide of the invention can be introduced into a cell via conventional breeding techniques.

Procedures for transforming both eukaryotic and prokaryotic organisms are well known and routine in the art and are described throughout the literature (*See*, for example, Jiang et al. 2013. Nat. Biotechnol. 31:233-239; Ran et al. Nature Protocols 8:2281-2308 (2013))

A nucleotide sequence therefore can be introduced into a host organism or its cell in any number of ways that are well known in the art. The methods of the invention do not depend on a particular method for introducing one or more nucleotide sequences into the organism, only that they gain access to the interior of at least one cell of the organism. Where more than one nucleotide sequence is to be introduced, they can be assembled as part of a single nucleic acid construct, or as separate nucleic acid constructs, and can be located on the same or different nucleic acid constructs. Accordingly, the nucleotide sequences can be introduced into the cell of interest in a single transformation event, or in separate transformation events, or, alternatively, where relevant, a nucleotide sequence can be incorporated into a plant, for example, as part of a breeding protocol.

The present invention is directed to the making and use of synthetic CRISPR-Cas nucleic acid constructs for DNA editing, site-specific cleavage or nicking of a target DNA, and/or transcriptional control of a target DNA.

The present inventors have surprisingly discovered that mutations in the stem (e.g., lower stem, and optionally, upper stem and kink,) and nexus hairpin regions of the crRNA-tracrRNA duplexes can produce single guides, which when complexed with a Cas9 polypeptide (endogenous or exogenous), can modify the activity of the Cas9 polypeptide as compared to a corresponding WT single guide. Thus, in some embodiments, a mutated single guide (e.g., a synthetic nucleic acid construct)/Cas9 polypeptide complex binds to a target DNA and exhibits reduced cleaving of the target DNA by the Cas9 polypeptide of the mutated guide/Cas9 complex as compared to cleaving of the target DNA by the Cas9 polypeptide when it is complexed with the corresponding WT guide. In some embodiments, a mutated single guide/Cas9 complex that binds to a target DNA and exhibits reduced cleaving of the target DNA may be useful for, for example, transcriptional control of target DNA. In some embodiments, a mutated single guide (e.g., a synthetic nucleic acid construct)/Cas9 polypeptide complex binds to a target DNA and exhibits increased cleaving of the target DNA by the Cas9 polypeptide of the mutated guide/Cas9 complex as compared to cleaving of the target DNA by the Cas9 polypeptide when complexed with the corresponding WT guide. In some embodiments, a mutated single guide/Cas9 complex that binds to a target DNA and exhibits increased cleaving of the target DNA may be useful for, for example, DNA editing, DNA cleavage, site specific dsDNA cleavage, and/or site specific dsDNA nicking.

In one aspect of the invention a synthetic nucleic acid construct is provided, comprising: (a) a crRNA comprising a 3' region and a 5' region, wherein the 3' region comprises at least about 10 consecutive nucleotides of a CRISPR repeat, and/or a functional fragment of the CRISPR repeat, and the 5' region comprises at least about 20 consecutive nucleotides of a spacer sequence located immediately upstream of the repeat; (b) a tracrRNA comprising a 5' and a 3' region, wherein at least a portion of the 5' region of the tracrRNA is complementary to the 3' region (i.e., CRISPR repeat) of the crRNA, wherein, when the 5' region of the tracrRNA that is complementary to the 3' region of the crRNA hybridizes to the 3' region of the crRNA, the synthetic nucleic acid construct forms secondary structures from 5' to 3' of: (i) a stem, the stem comprising a duplex between the 5' end of the tracrRNA and the repeat of the crRNA, and optionally, the stem further comprising a kink or a bulge; (ii) a nexus hairpin; (iii) at least one terminal hairpin; and (c) optionally, a linker that when present links the 3'end of the crRNA to the 5' end of the tracrRNA (i.e., linking the 3' end and 5' end of the stem), wherein the construct comprises an insertion, substitution, and/or deletion of about one to about five nucleotides or base pairs (e.g., 1, 2, 3, 4, 5 nucleotides or base pairs) in the nexus hairpin as compared to a wild type Type II crRNA:tracrRNA duplex; and/or an insertion, substitution and/or deletion of about one to about nine nucleotides and/or base pairs (e.g., 1, 2, 3, 4, 5, 6,7, 8, 9 nucleotides or base pairs) in the stem as compared to a wild type Type II crRNA:tracrRNA duplex.

In a further aspect, the present invention provides a protein-RNA complex, comprising: (a) a Cas9 polypeptide; and (b) a synthetic nucleic acid construct comprising: (a) a crRNA comprising a 3' region and a 5' region, wherein the 3' region comprises at least about 10 consecutive nucleotides of a CRISPR repeat, and/or a functional fragment of the CRISPR repeat, and the 5' region comprises at least about 20 consecutive nucleotides of a spacer sequence located immediately upstream of the repeat; (b) a tracrRNA comprising a 5' and a 3' region, wherein at least a portion of the 5' region of the tracrRNA is complementary to the 3' region (i.e., CRISPR repeat) of the crRNA, wherein, when the 5' region of the tracrRNA that is complementary to the 3' region of the crRNA hybridizes to the 3' region of the crRNA, the synthetic nucleic acid construct forms secondary structures from 5' to 3' of: (i) a stem, the stem comprising a duplex between the 5' end of the tracrRNA and the repeat of the crRNA, and optionally, the stem further comprising a kink or a bulge; (ii) a nexus hairpin; (iii) at least one terminal hairpin; and (c) optionally, a linker that when present links the 3'end of the crRNA to the 5' end of the tracrRNA (i.e., linking the 3' end and 5' end of the stem), wherein the construct comprises an insertion, substitution, and/or deletion of about one to about five nucleotides or base pairs (e.g., 1, 2, 3, 4, 5 nucleotides or base pairs) in the nexus hairpin as compared to a wild type Type II crRNA:tracrRNA duplex; and/or an insertion, substitution and/or deletion of about one to about nine nucleotides and/or base pairs (e.g., 1, 2, 3, 4, 5, 6,7, 8, 9 nucleotides or base pairs) in the stem as compared to a wild type Type II crRNA:tracrRNA duplex.

In some embodiments of the present invention, at least one terminal hairpin comprises at least two terminal hairpins, 1 to 2 terminal hairpins, 1 to 3 terminal hairpins, 1 terminal hairpin, 2 terminal hairpins, or 3 terminal hairpins.

In some embodiments, a linker between the crRNA and the tracrRNA (at the stem region) is present. In some embodiments, a linker may comprise at least 3 nucleotides (e.g., 3, 4, 5, 6 nucleotides). In some embodiments, a linker may comprise about 3 to about 6 nucleotides (e.g., 3, 4, 5, or 6 nucleotides) or about 3 to about 5 nucleotides (e.g., 3, 4, or 5 nucleotides).

In some embodiments of the present invention, a modification (e.g., a substitution, deletion or insertion) of one or both nucleotides of a base pair constitutes a single modification (i.e., counted as one).

In some embodiments, an insertion, substitution, and/or deletion in the nexus hairpin can comprise an insertion, substitution, and/or deletion of about 1 to 2, 1 to 3, 1 to 4, 1 to 5, 2 to 3, 2 to 4, 2 to 5, 3 to 4, 3 to 5, 4 to 5 nucleotides and/or base pairs, or any range or value therein.

In some embodiments, an insertion, substitution, and/or deletion in the stem (e.g., lower stem, and/or lower stem, kink and upper stem) can comprise an insertion, substitution, and/or deletion of about 1 to 2, 1 to 3, 1 to 4, 1 to 5, 1 to 6, 1 to 7, 1 to 8, 2 to 3, 2 to 4, 2 to 5, 2 to 6, 2 to 7, 2 to 8, 2 to 9, 3 to 4, 3 to 5, 3 to 6, 3 to7, 3 to 8, 3 to 9, 4 to 5, 4 to 6, 4 to 7, 4 to 8, 4 to 9, 5 to 6, 5 to 7, 5 to 8, 5 to 9, 6 to 7, 6 to 8, 6 to 9, 7 to 8, 7 to 9, 8 to 9 nucleotides and/or base pairs, or any range or value therein.

In some embodiments, an insertion, substitution and/or deletion may increase the GC content of the synthetic nucleic acid construct as compared to a wild type Type II crRNA:tracrRNA duplex. In some embodiments, 1, a substitution may comprise replacing a complementary base pair with a pair of non-complementary (unmatched) nucleotides, thereby introducing a bulge. In some embodiments, a substitution may comprise replacing a complementary base pair with a different complementary base pair, thereby maintaining a duplex. In some embodiments, a substitution may comprise replacing a pair of non-complementary nucleotides with a different pair of non-complementary nucleotides, thereby maintaining a bulge. In some embodiments, a substitution may comprise replacing a pair of non-complementary nucleotides with a pair of complementary nucleotides, thereby modifying a bulge.

In some embodiments, a substitution may comprise a pyrimidine nucleotide replaced by another pyrimidine nucleotide, a purine nucleotide replaced by a purine nucleotide, a purine nucleotide replaced by a pyrimidine nucleotide, and/or a pyrimidine nucleotide replaced by a purine nucleotide.

In some embodiments, an insertion may increase the distance between the stem and the nexus. In some embodiments, a substitution and/or insertion may produce a bulge in the stem. In some embodiments, when a kink is present in the stem region, a substitution and/or insertion may increase or decrease the size of the kink (e.g., size may be increase when additional unpaired bases are generated, and size may be decreased when additional paired bases are generated). In some embodiments, a deletion may shorten or eliminate at least a portion of a stem. In some embodiments, a deletion may shorten the nexus hairpin region. In some embodiments, an insertion may lengthen the nexus hairpin region.

As is well known in the art, Cas9 polypeptides are multifunctional proteins that bind DNA (e.g., target DNA), RNA (guide RNA) and specific nucleotide sequences called protospacer adjacent motifs (PAM), in addition to comprising nuclease/nickase activity (RuvC and HNH motifs) that allows them to cut each strand of a double stranded nucleic acid. In some embodiments of the invention, a CRISPR Cas9 polypeptide may be a Cas9 polypeptide from *Lactobacillus* spp., *Bifidobacterium* spp., *Kandleria* spp., *Leuconostoc* spp., *Oenococcus* spp., *Pediococcus* spp., *Streptococcus* spp., *Weissella* spp., and/or *Olsenella* spp. As noted above, Cas9 polypeptides can recognize and bind to the PAM sequences that are located on the target DNA. Cas9 polypeptides exhibit specificity for the particular PAM sequence that they recognize and bind to.

In some embodiments, a Cas9 polypeptide can be encoded by a nucleotide sequence of any of **SEQ ID NOs:294-388,** may be a polypeptide comprising the amino acid sequence of **SEQ ID NOs:194 -293,** or encoded by a nucleotide sequence encoding a polypeptide comprising the amino acid sequence of **SEQ ID NOs:194-293.**

The structure of Cas9 polypeptides is described in the art and understood to comprise a nuclease lobe (NUC) and a recognition lobe (REC), with the NUC lobe interacting with the PAM and target DNA, while the REC lobe interacts with and binds to the sgRNA (crRNA-tracrRNA) (see, Barrangou, R. Science 344:707-708 (2104), generally and figure presented therein. It is between the groove located between the two lobes that the sgRNA-target DNA heteroduplex is formed. *Id.* Further, as noted above, the HNH and RuvC motifs of a Cas9 polypeptide have been characterized (Sapranauskas et al. Nucleic Acids Res. 39:9275-9282 (2011)). Additional details regarding the structure of Cas9 polypeptides and their interaction with tracrRNA and crRNA (sgRNAs) can be found in Nishimasu et al. (Cell 156(5):935-949 (2014)). Here, they provide the crystal structure of Cas9 in complex with guide RNA and target RNA as well as schematics of the sgRNA:target DNA complex and the structure of the Cas9 polypeptide. Thus, the structure of the Cas9 polypeptide is well characterized with the various functions of the protein associated with defined structures within the polypeptide.

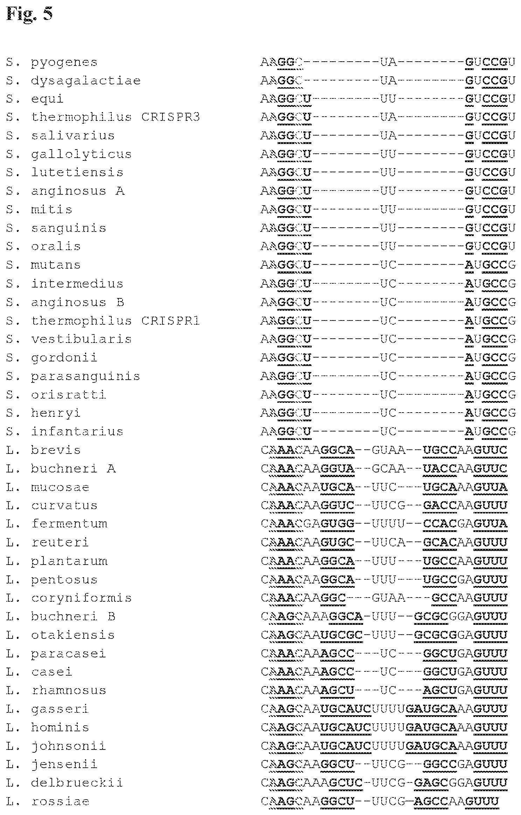

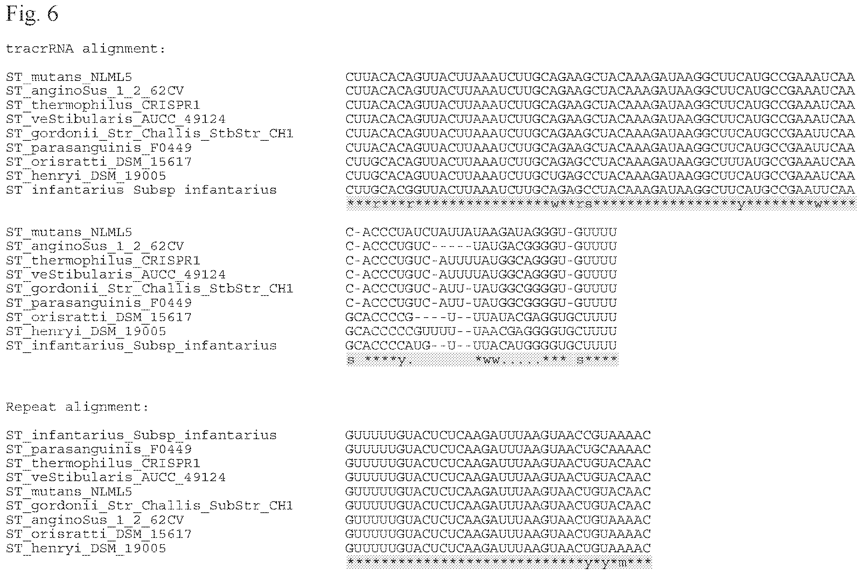

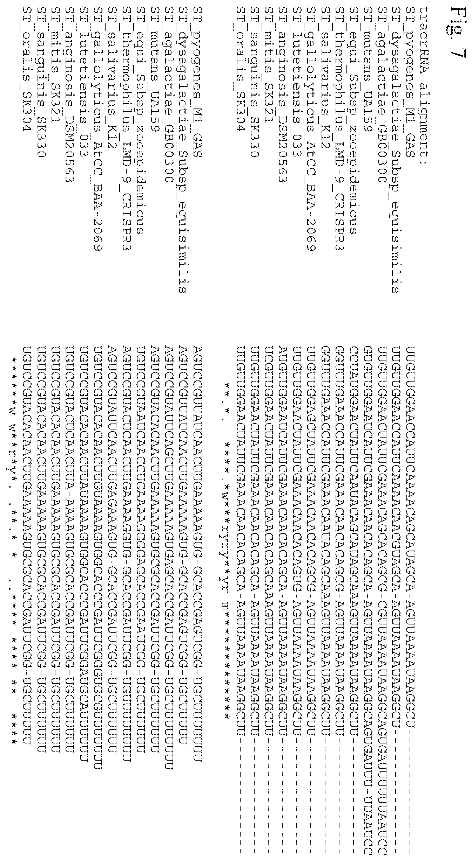

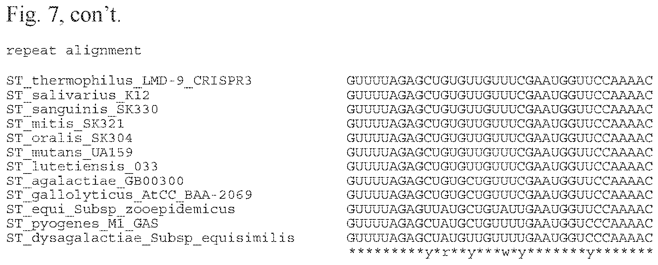

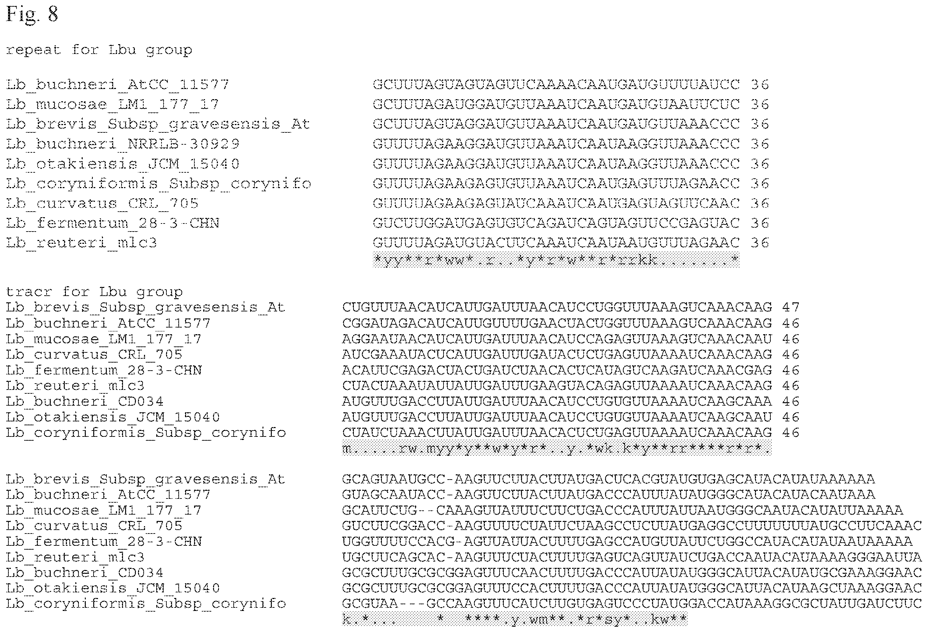

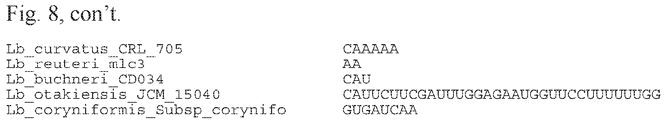

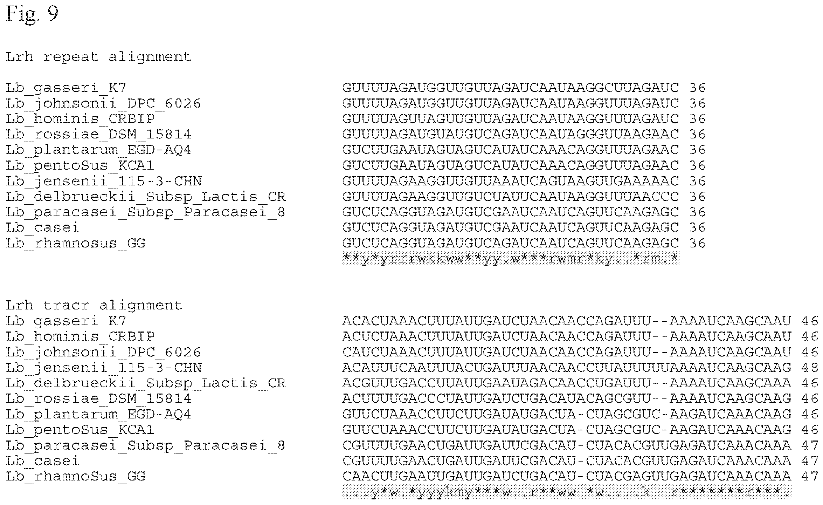

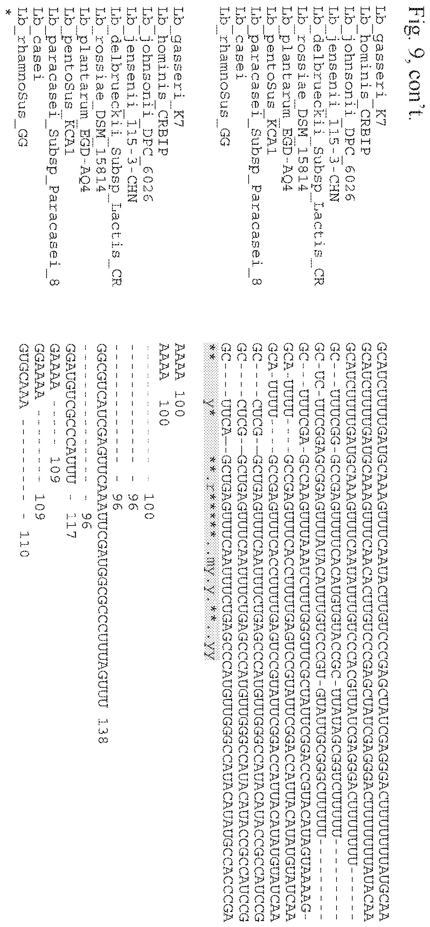

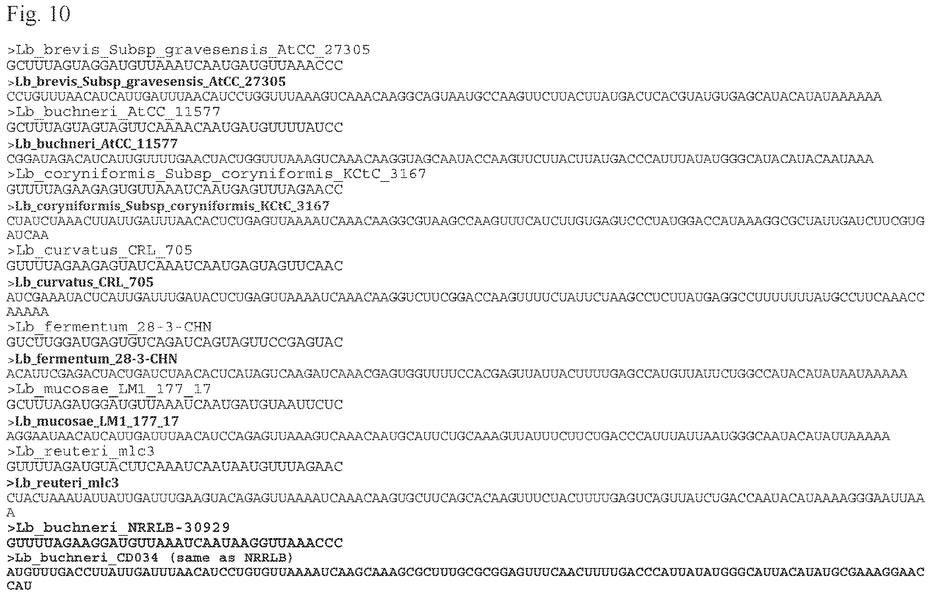

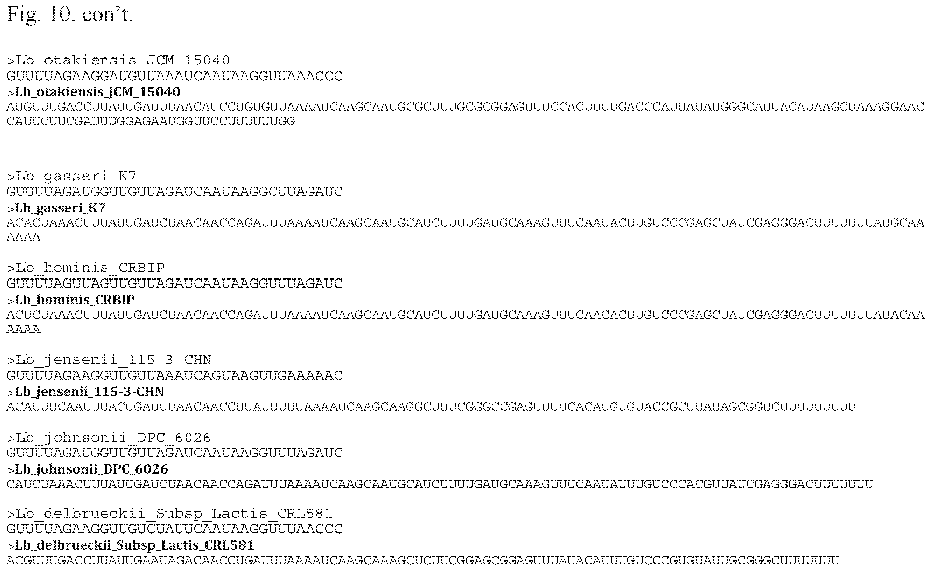

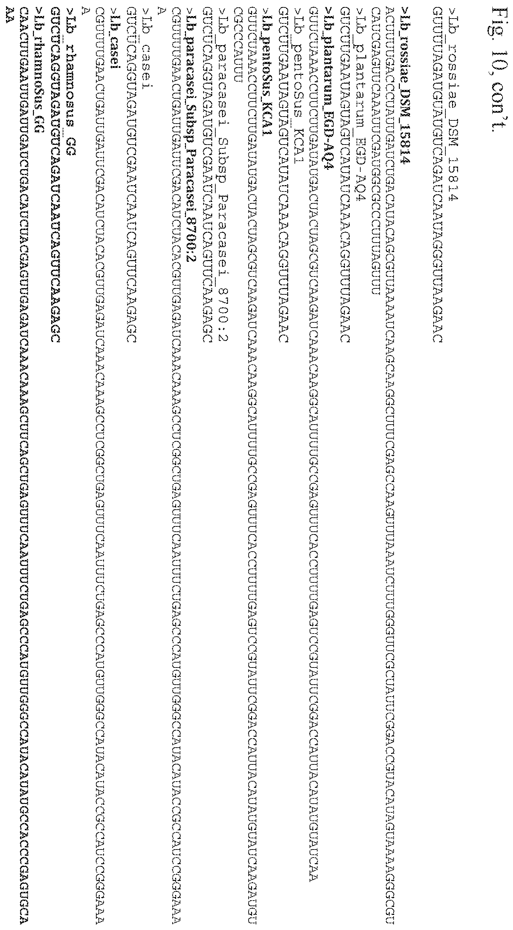

In some embodiments, a crRNA of this invention, comprising a 3' region and a 5' region, can further comprise a CRISPR repeat located upstream of the spacer sequence, wherein the CRISPR repeat comprises, consists essentially, or consists of at least about 10 consecutive nucleotides (e.g., about 10, 11, 12, 13, 14, 15, 16, 17, 18, 19, 20, 21, 22, 23, 24, 25, 26, 27, 28, 29, 30 or more nucleotides) of CRISPR repeat from a Type II CRISPR-Cas system, for example, from *Lactobacillus* spp., *Bifidobacterium* spp., *Kandleria* spp., *Leuconostoc* spp., *Oenococcus* spp., *Pediococcus* spp., *Streptococcus* spp., *Weissella* spp., and/or *Olsenella* spp. (e.g., **SEQ ID NOs:1-98,** or a functional fragment thereof, which are modified to produce a modified synthetic guide as described herein). Therefore, in representative embodiments, a crRNA can comprise, consist essentially of, or consist of (from 5' to 3') a spacer sequence - a CRISPR repeat, or a CRISPR repeat - a spacer sequence - a CRISPR repeat. As used herein, a "functional fragment" of a crRNA means a portion of said crRNA which binds to the corresponding tracrRNA and/or a portion of which is recognized and bound to a corresponding Cas9 polypeptide. **Fig. 5** shows example CRISPR repeat sequence alignments with conserved and consensus nucleotides specified at the bottom of each family, with Sth3 (top), Sth1 (middle) and Lb (bottom) families, thereby providing structural references for crRNA fragments for use with this invention. **Figs. 6-9** provide additional alignments of example CRISPR repeat and tracrRNA sequence for *Lactobacillus* and *Streptococcus* species/strains useful with this invention. Additional example repeat sequences and tracrRNA sequence from various organisms are provided in **Fig. 10****.**

A "spacer sequence" as used herein means a sequence that is upstream (5') of a repeat in a crRNA. Alternatively, when the crRNA comprises two repeats (i.e., a first and a second repeat) the spacer sequence is located between the two repeats (i.e., the spacer sequence is located 3' of the first repeat and 5' of the second repeat). Generally, the spacer sequence comprises a polynucleotide sequence that is complementary to a target DNA and/or an invasive foreign (e.g., heterologous) DNA (e.g., a nucleotide sequence from a bacteriophage, plasmid or chromosome that is foreign to, for example, a bacterium or an archaeon). The spacer sequence can be at least 70% complementary (e.g., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% homologous) to the target DNA. In representative embodiments, the spacer sequence is 100% complementary to the target DNA. In other embodiments, the complementarity of the 3' region of the spacer sequence to the target or DNA is 100% but is less than 100% in the 5' region of the spacer. Thus, for example, the first 7, 8, 9, 10, 11, 12, 13, 14, 15, 16, and the like, nucleotides in the 3' region of a 20 nucleotide spacer sequence (seed sequence) can be 100% complementary the target DNA, while the remaining nucleotides in the 5' region of the spacer sequence are at least about 70% complementary to the target DNA. In representative embodiments, the first 12 nucleotides of the spacer sequence can be 100% complementary to the target DNA, while the remaining nucleotides in the 5' region of the spacer sequence be at least about 70% complementary to the target DNA.

In some embodiments, a repeat for use with this invention can comprise, consist essentially of, or consist of a repeat from *Lactobacillus* spp., *Bifidobacterium* spp., *Kandleria* spp., *Leuconostoc* spp., *Oenococcus* spp., *Pediococcus* spp., *Streptococcus* spp., *Weissella* spp., and/or *Olsenella* spp. In some embodiments, a crRNA repeat from *Lactobacillus* spp., *Bifidobacterium* spp., *Kandleria* spp., *Leuconostoc* spp., *Oenococcus* spp., *Pediococcus* spp., *Streptococcus* spp., *Weissella* spp., and/or *Olsenella* spp. comprises, consists essentially of, or consists of a nucleotide sequence of any of **SEQ ID NOs:1-98** or a functional fragment thereof, which may be modified to produce a synthetic repeat for use in a synthetic crRNA or a synthetic crRNA:tracrRNA guide as described herein.

In some embodiments, the 5' region of the tracrRNA of the synthetic nucleic acid construct can comprise, consist essentially of, or consist of at least about 20 nucleotides (e.g., 20, 21, 22, 23, 24, 25, 26, 27, 28, 29, 30, 31, 32, 33, 34, 35, 36 or more consecutive nucleotides) complementary to the at least 20 consecutive nucleotides of the 3' region of the crRNA.

The 5' region of the tracrRNA is described herein as complementary to the 3' region (repeat) of the crRNA. In some embodiments, "complementary" means having at least about 70% or more (e.g., about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%) homology to the 3' repeat of the crRNA. Thus, for example, the 5' region of a tracrRNA that is complementary to about 20 consecutive nucleotides of a crRNA can have 100% complementarity to about 14 out of 20 consecutive nucleotides of the crRNA repeat. In representative embodiments, the 5' region of a tracrRNA that is complementary to a 20 consecutive nucleotides of a crRNA can have 100% complementarity to at least 7 consecutive nucleotides (e.g., 7, 8, 9, 10, 11, 12, 13, 14, 15, 16, 17, 18, 19, 20 nucleotides) of the crRNA repeat. Example tracrRNA nucleotide sequences useful with this invention can be a tracrRNA from any Type II CRISPR Cas system including but not limited to **SEQ ID NOs:99-193,** which may be modified to produce a synthetic tracrRNA or a synthetic crRNA:tracrRNA guide as described herein.

The present invention further provides expression cassettes and/or vectors comprising or encoding the nucleotide sequences (e.g., a synthetic nucleic acid construct, protein-RNA construct; crRNA, tracrRNA, Cas9) of this invention.

The present invention additionally provides a cell comprising synthetic nucleic acid constructs and/or protein-RNA complexes of this invention. A cell can be from any organism useful with this invention including, but not limited to, a plant cell, bacteria cell, fungal cell, an animal cell, mammalian cell, insect cell, or archaeon cell. In some embodiments, the cell can be, for example, from *Homo sapiens, Drosophila melanogaster, Mus musculus, Rattus norvegicus, Caenorhabditis elegans*, *Saccharomyces cerevisiae, Zea mays,* or *Arabidopsis thaliana,* and the like.

In some embodiments, the present invention provides a method of producing a modified Type II crRNA:tracrRNA construct, comprising: (a) inserting, substituting, and/or deleting about one to about five nucleotides or base pairs in the nexus hairpin of a Type II crRNA:tracrRNA; and/or (b) inserting, substituting, and/or deleting about one to about nine nucleotides and/or base pairs in a stem of the Type II crRNA:tracrRNA, wherein the crRNA comprises a 3' region and a 5' region, the 3' region comprising at least about 10 consecutive nucleotides of a CRISPR repeat, and/or a functional fragment of the repeat, and the 5' region comprises at least about 20 consecutive nucleotides of a spacer sequence located immediately upstream of the repeat, and the tracrRNA comprises a 5' and a 3' region, and at least a portion of the 5' region of the tracrRNA is complementary to the 3' region (i.e., CRISPR repeat) of the crRNA, further wherein, when the 5' region of the tracrRNA that is complementary to the 3' region of the crRNA hybridizes to the 3' region of the crRNA, the Type II crRNA:tracrRNA forms secondary structures from 5' to 3' of: (i) a stem, the stem comprising a duplex between the 5' end of the tracrRNA and the repeat of the crRNA, and optionally, the stem further comprising a kink or a bulge; (ii) a nexus hairpin; (iii) at least one terminal hairpin; and (c) optionally, a linker that when present links the 3'end of the crRNA to the 5' end of the tracrRNA (i.e., linking the 3' end and 5' end of the stem), thereby producing a modified Type II rRNA:tracrRNAs construct. In some embodiments, the present invention provides a modified Type II rRNA:tracrRNAs construct (e.g., a synthetic nucleic acid construct) produced by the methods of the invention.

In some embodiments, the present invention provides a method of modifying the activity of a Cas9 polypeptide, comprising complexing the Cas9 polypeptide with a synthetic nucleic acid construct of the invention, thereby modifying the activity of a Cas9 polypeptide as compared to a Cas9 polypeptide complexed with a wild type Type II crRNA:tracrRNA duplex. In some aspects of the present invention, the activity of the Cas9 polypeptide when complexed with the synthetic nucleic acid construct is increased (e.g., increased cleavage/killing) as compared to a Cas9 polypeptide complexed with a wild type Type II crRNA:tracrRNA duplex. In some aspects of the present invention, the activity of the Cas9 polypeptide when complexed with the synthetic nucleic acid construct is decreased (e.g., reduced or no cleavage/killing) as compared to a Cas9 polypeptide complexed with a corresponding wild type Type II crRNA:tracrRNA duplex.

In some embodiments, the present invention provides a method of controlling transcription of a target DNA, comprising: contacting the target DNA with a synthetic nucleic acid construct of the invention and/or an expression cassette and/or vector comprising the synthetic nucleic acid construct, in the presence of a Cas9 polypeptide, wherein the synthetic nucleic acid construct and Cas9 polypeptide form a complex that binds to the target DNA, thereby controlling the transcription of the target DNA. Thus, in some embodiments, a synthetic guide of the present invention, in the presence of a Cas9 polypeptide, may bind the target DNA but prevents cleavage, thereby controlling the transcription of the target DNA. In some embodiments, the Cas 9 polypeptide is an endogenous Cas9 polypeptide. In some embodiments, a Cas9 polypeptide may be provided with the synthetic guide of the present invention. Thus, in some embodiments, the present invention provides a method of controlling transcription of a target DNA, comprising: contacting the target DNA with a protein-RNA complex of the present invention, and/or an expression cassette and/or a vector comprising a synthetic nucleic acid construct and encoding a Cas9 polypeptide of the protein-RNA complex, wherein the protein RNA complex binds to the target DNA, thereby controlling the transcription of the target DNA.

"Transcriptional control" as used herein means modulating expression of a target DNA (i.e., activation (increasing) and/or repression (decreasing) expression of the target DNA. Repression of expression can be accomplished by, for example, using a Cas9 polypeptide (endogenous or heterologous) with a synthetic nucleic acid construct of the invention or a protein-RNA complexes of the invention, which bind to the target nucleic acid and interfere with or repress expression of the target nucleic acid.

In some embodiments, a method for site-specific cleavage of a target DNA is provided, comprising: contacting the target DNA with a synthetic nucleic acid construct of the present invention, and/or an expression cassette and/or a vector comprising a synthetic nucleic acid construct of the invention, in the presence of a Cas9 polypeptide, thereby producing a site specific cleavage of the target DNA in a region defined by complementary binding of the spacer sequence of the crRNA of said synthetic nucleic acid construct to the target DNA.

In some embodiments, a method for site-specific cleavage of a target DNA is provided, comprising: contacting the target DNA with a protein-RNA complex of the present invention, and/or an expression cassette and/or a vector comprising a synthetic nucleic acid construct of the invention and encoding a Cas9 polypeptide of the protein-RNA complex, thereby producing a site specific cleavage of the target DNA in a region defined by complementary binding of the spacer sequence of the crRNA of the protein-RNA complex to the target DNA.

In some embodiments, a method of editing a target DNA is provided, comprising: contacting the target DNA with a synthetic nucleic acid construct of the present invention, and/or with an expression cassette and/or a vector comprising the synthetic nucleic acid construct, in the presence of a Cas9 polypeptide, wherein the synthetic nucleic acid construct binds to the target DNA, thereby editing the target DNA.

In some embodiments, the present invention provides a method of editing a target DNA, comprising: contacting the target DNA with a protein-RNA complex of the present invention, and/or an expression cassette and/or a vector comprising a synthetic nucleic acid construct and encoding a Cas9 polypeptide of the protein-RNA complex, wherein the protein-RNA complex binds to the target DNA, thereby editing the target DNA.

In some embodiments, contacting a target DNA with a synthetic nucleic acid construct of the present invention and Cas9 polypeptide and/or a protein-RNA complex of the present invention comprises contacting the target DNA with synthetic nucleic acid construct of the present invention and/or a protein-RNA complex encoded or comprised in an expression cassette or vector. Accordingly, in some embodiments, an expression cassette and/or vector can be constructed to produce a synthetic nucleic acid construct of the invention (e.g., mutated tracrRNA-crRNA (sgRNA)) and optionally express a Cas9 polypeptide (e.g., a heterologous/exogenous Cas9). In some embodiments, a Cas9 polypeptide can be provided in combination with an expression cassette or vector comprising or encoding a sgRNA. In some embodiments, no expression cassette or vector is utilized, instead a target DNA can be contacted with a protein-RNA complex of the invention itself.

In some embodiments of the invention, a method for site-specific nicking of a (+) strand of a double stranded target DNA provided, comprising: contacting the double stranded target DNA with a protein-RNA complex of the present invention, and/or an expression cassette and/or a vector comprising a synthetic nucleic acid construct and encoding a Cas9 polypeptide of the protein-RNA complex, wherein the Cas9 polypeptide, comprises a point mutation in an RuvC active site motif, and the Cas9 polypeptide cleaves the (+) strand of the double stranded DNA, thereby producing a site-specific nick in said double stranded target DNA.

In some embodiments, the invention provides a method for site-specific nicking of a (-) strand of a double stranded target DNA, comprising: contacting the double stranded target DNA with a protein-RNA complex of the present invention, and/or an expression cassette and/or a vector comprising a synthetic nucleic acid construct and encoding a Cas9 polypeptide of the protein-RNA complex, wherein the Cas9 polypeptide, comprises a point mutation in an HNH active site motif, and the Cas9 polypeptide cleaves the (-) strand of the double stranded DNA, thereby producing a site-specific nick in said double stranded target DNA.

A Cas9 polypeptide or nuclease useful with this invention can be any Cas9 polypeptide. A Cas9 polypeptide can be from, for example, *Lactobacillus* spp., *Bifidobacterium* spp., *Kandleria* spp., *Leuconostoc* spp., *Oenococcus* spp., *Pediococcus* spp., *Streptococcus* spp., *Weissella* spp., and/or *Olsenella* spp. In some embodiments, a Cas9 polypeptide can comprise, consist essentially of, or consist of an amino acid sequence having at least about 80% (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) identity to a polypeptide comprising the amino acid sequence of **SEQ ID NOs:194-293.**

In some embodiments, once a target DNA is cleaved, it can then be modified by repair mechanisms as known in the art, notably non-homologous end-joining (NHEJ) and homology-directed repair (HDR). Thus, in some embodiments, a donor DNA can be provided for assisting in repair, such as a dsDNA template for homologous recombination in HDR-mediated repair.

In some embodiments, a Cas9 polypeptide can be codon optimized for the organism comprising the target DNA as described herein and as known in the art. Non-limiting examples of the types of organisms useful with this invention include plants, bacteria, fungi, mammals, insects, or archaea. In some embodiments, a polypeptide, and/or a functional fragment thereof, having at least about 80% identity with a Cas9 polypeptide can be codon optimized to be expressed in the organism comprising the target DNA.

In some embodiments, a repeat, a tracrRNA sequence, a Cas9 polypeptide or and/or a Cas9 nucleotide sequence can be from *Lactobacillus* spp., *Bifidobacterium* spp., *Kandleria* spp., *Leuconostoc* spp., *Oenococcus* spp., *Pediococcus* spp., *Streptococcus* spp., *Weissella* spp., or *Olsenella* spp. In representative embodiments, a repeat, a tracRNA sequence, a Cas9 polypeptide or and/or a Cas9 nucleotide sequence can be from *Bifidobacterium bombi*, *Bifidobacterium dentium* LMG 11045, *Bifidobacterium merycicum* LMG 11341, *Kandleria vitulina* DSM 20405, *Lactobacillus agilis* DSM 20509, *Lactobacillus animalis* DSM 20602, *Lactobacillus apodemi* DSM 16634, *Lactobacillus brevis subsp gravensis* ATCC 27305, *Lactobacillus buchneri* CD034, *Lactobacillus buchneri* DSM 20057, Lactobacillus cacaonum DSM 21116, *Lactobacillus casei* str Zhang, *Lactobacillus ceti* DSM 22408, *Lactobacillus coryniformis subsp coryniformis* KCTC 3167, *Lactobacillus coryniformis torquens, Lactobacillus crispatus* FB049-03, *Lactobacillus curvatus* CRL 705, *Lactobacillus delbrueckii subsp lactis* CRL 581, *Lactobacillus delbrueckii jakobsenii* DSM 26046, *Lactobacillus diolivorans* DSM 14421, *Lactobacillus farciminis* DSM 20184, *Lactobacillus fermentum* DSM 20055, *Lactobacillus floricola* DSM 23037A, *Lactobacillus floricola* DSM 23037B, *Lactobacillus fuchuensis* DSM 14340, *Lactobacillus futsaii* JCM 17355, *Lactobacillus gasseri K7, Lactobacillus graminis* DSM 20719, *Lactobacillus hammesii* DSM *16381, Lactobacillus hominis* CRBIP, *Lactobacillus hordei* DSM 19519A, *Lactobacillus hordei* DSM 19519B, *Lactobacillus jensenii* DSM 20557, *Lactobacillus johnsonii* DPC 6026, *Lactobacillus lindneri* DSM 20690, *Lactobacillus mali* ATCC 27304, *Lactobacillus mindensis* DSM 14500, *Lactobacillus mucosae* DSM 13345, *Lactobacillus namurensis* DSM *19117, Lactobacillus nantensis* DSM 16982, *Lactobacillus nodensis* DSM 19682A, *Lactobacillus nodensis* DSM 19682B, *Lactobacillus oligofermentans* DSM 15707, *Lactobacillus otakiensis* DSM 19908, *Lactobacillus ozensis* DSM 23829, *Lactobacillus paracasei subsp paracasei* 8700:2, *Lactobacillus paracasei subsp tolerans* Lpl7, *Lactobacillus paracollinoides* DSM 15502, *Lactobacillus parakefiri* DSM 10551, *Lactobacillus pentosus* KCA1, *Lactobacillus pentosus* IG1, *Lactobacillus plantarum* EGD-AQ4, *Lactobacillus psittaci* DSM 15354, Lactobacillus rennini DSM 20253, Lactobacillus reuteri mlc3, *Lactobacillus rhamnosus* GG, *Lactobacillus rossiae* DSM 15814, *Lactobacillus ruminis* ATCC 25644, *Lactobacillus sakei carnosus* DSM 15831, *Lactobacillus salivarius* TCC 118, *Lactobacillus sanfranciscensis* DSM 20451, *Lactobacillus saniviri* DSM 24301, *Lactobacillus senmaizukei* DSM 21775, *Lactobacillus tucceti* DSM 20183, *Lactobacillus versmoldensis* DSM 14857, *Lactobacillus zymae* DSM 19395, *Leuconostoc gelidum* JB7, *Leuconostoc pseudomesenteroides* 4882, *Oenococcus kitaharae* DSM 17330, *Pediococcus inopinatus* DSM 20285, *Pediococcus* lolii DSM 19927, *Pediococcus parvulus* DSM 20332A, *Pediococcus parvulus* DSM 20332B, *Pediococcus stilesii* DSM 18001, *Streptococcus agalactiae* GB00300, *Streptococcus gallolyticus* ATCC BAA-2069, *Streptococcus henryi* DSM 19005, *Streptococcus mutans* NLML5, *Streptococcus oralis* SK304, Streptococcus anginosus 1_2_62CV, *Streptococcus anginosus* DSM 20563, *Streptococcus dysagalactiae subsp equisimilis, Streptococcus equi subsp zooepidemicus, Streptococcus gordonii Challis* substr CH1, *Streptococcus infantarius subsp infantarius, Streptococcus intermedius* B196, *Streptococcus lutetiensis* 033, *Streptococcus mitis* SK321, *Streptococcus mutans* UA 159, *Streptococcus orisratti* DSM 15617, *Streptococcus parasanguinis* F0449, *Streptococcus salivarius* K12, *Streptococcus sanguinis* SK330, *Streptococcus vestibularis* ATCC 49124, *Lactobacillus composti* DSM 18527, *Lactobacillus concavus* DSM 17758, *Lactobacillus secaliphilus* DSM 17896, *Weissella halotolerans* DSM 20190, *Weissella kandleri* DSM 20593, and/or *Olsenella uli* or any combination thereof.

In some embodiments, combinations of repeats, tracrRNA sequences, Cas9 polypeptide sequences and Cas9 nucleotide sequences that may be useful with this invention are set forth in **Table 1** below.

**Table 1. Combinations of repeats, tracRNA sequences, and Cas9 polypeptides and nucleic acid sequences useful with the invention.**

| **Repeat (SEQ ID NO)** | **TracRNA sequence (SEQ ID NO)** | **Cas9 polypeptide sequence (SEQ ID NO)** | **Cas9 nucleotide sequence (SEQ ID NO)** | **PAM Sequence** |
|---|---|---|---|---|
| 1 | | 194 | 294 | nnC |
| 2 | 99 | 195 | 295 | |
| 3 | 100 | 196 | 296 | nGG |
| 4 | 101 | 197 | 297 | |
| 5 | 102 | 198 | 298 | nGG |
| 6 | 103 | 199 | 299 | |
| 7 | 104 | 200 | 300 | |
| 8 | 105 | 201 | 301 | nnAAAA |
| 9 | 106 | 202 | 303 | |
| 10 | 107 | 203 | 302 | nnnAnnA |
| 11 | 108 | 204 | 304 | |
| 12 | 109 | 205 | 305 | nGA |
| 13 | 110 | 206 | 306 | |
| 14 | 111 | 207 | | |
| 15 | | 208 | 307 | |
| 16 | 112 | 209 | | |
| 17 | 113 | 210 | 308 | |
| 18 | 114 | 211 | 309 | |
| 19 | 115 | 212 | 310 | |
| 20 | 116 | 213 | 311 | |
| 21 | 117 | 214 | 312 | |
| 22 | 118 | 215 | 313 | |
| 23 | 119 | 216 | 314 | |
| 24 | 120 | 217 | 315 | |
| 25 | 121 | 218 | 316 | |
| 26 | 122 | 219 | 317 | |
| 27 | 123 | 220 | 318 | nTAA |
| 28 | 124 | 221 | 319 | |
| 29 | 125 | 222 | 320 | |
| 30 | 126 | 223 | 321 | |
| 31 | 127 | 224 | 322 | |
| 32 | 128 | 225 | 323 | |
| 33 | 129 | 226 | 324 | nGG |
| 34 | 130 | 227 | 325 | |
| 35 | 131 | 228 | 326 | |
| 36 | 132 | 229 | 327 | nnAnAA |
| 37 | 133 | 230 | 328 | |
| 38 | 134 | 231 | 329 | |
| 39 | 135 | 232 | 330 | |
| 40 | 136 | 233 | 331 | |
| 41 | 137 | 234 | 332 | |
| 42 | 138 | 235 | 333 | |
| 43 | 139 | 236 | 334 | |
| 44 | 140 | 237 | 335 | |
| 45 | 141 | 238 | 336 | |
| 46 | 142 | 239 | 337 | |
| 47 | 143 | 240 | 338 | |
| 48 | 144 | 241 | 339 | |
| 49 | 145 | 241 | 340 | |
| 50 | 146 | 243 | 342 | |
| 51 | 147 | 244 | 341 | nnG |
| 52 | 148 | 245 | 343 | |
| 53 | 149 | 246 | 344 | |
| 54 | 150 | 247 | 345 | |
| 55 | 151 | 248 | | nGAAA |
| 56 | 152/153 | 249/250 | 346 | |
| 57 | 154 | 251 | 347 | |
| 58 | 155 | 252 | 348 | |
| 59 | 156 | 253 | 349 | |
| 60 | 157 | 254 | 350 | |
| 61 | 158 | 255 | 351 | |
| 62 | 159 | 256 | 352 | |
| 63 | 160 | 257 | 353 | |
| 64 | 161 | 258 | 354 | |
| 65 | 162 | 259 | 355 | |
| 66 | 163 | 260 | 356 | |
| 67 | 164 | 261 | 357 | |
| 68 | 165 | 262 | 358 | |
| 69 | 166 | 263 | 359 | nTG |
| 70 | 167 | 264 | 360 | |
| 71 | 168 | 265 | 361 | |
| 72 | 169 | 266 | 362 | AGA |
| 73 | 170 | 267 | 363 | nnAAC |
| 74 | 171 | 268 | 364 | |
| 75 | 172 | 269 | 365 | |
| 76 | 173 | 270 | 366 | |
| 77 | 174 | 271 | | |
| 78 | 175 | 272 | 367 | |
| 79 | 176 | 273 | 368 | |
| 80 | 177 | 274 | 369 | |
| 81 | 178 | 275 | 370 | |
| 82 | 179 | 276 | 371 | |
| 83 | 180 | 277 | 372 | |
| 84 | 181 | 278 | 373 | |
| 85 | 182 | 279 | 374 | |
| 86 | 183 | 280 | 375 | |
| 87 | 184 | 281 | 376 | |
| 88 | 185 | 282 | 377 | |
| 89 | 186 | 283 | 378 | |
| 90 | 187 | 284 | 379 | |
| 91 | 188 | 285 | 380 | |
| 92 | 189 | 286 | 381 | |
| 93 | 190 | 287 | 382 | |
| 94 | 191 | 288 | 383 | |
| 95 | 192 | 289 | 384 | |
| 96 | 193 | 290 | 385 | |
| 97 | | 291 | 386 | |
| 98 | | 292 | 387 | |

The invention will now be described with reference to the following examples. It should be appreciated that these examples are not intended to limit the scope of the claims to the invention, but are rather intended to be exemplary of certain embodiments. Any variations in the exemplified methods that occur to the skilled artisan are intended to fall within the scope of the invention.

### EXAMPLES

### Example 1. Modifying CRISPR-Cas guides

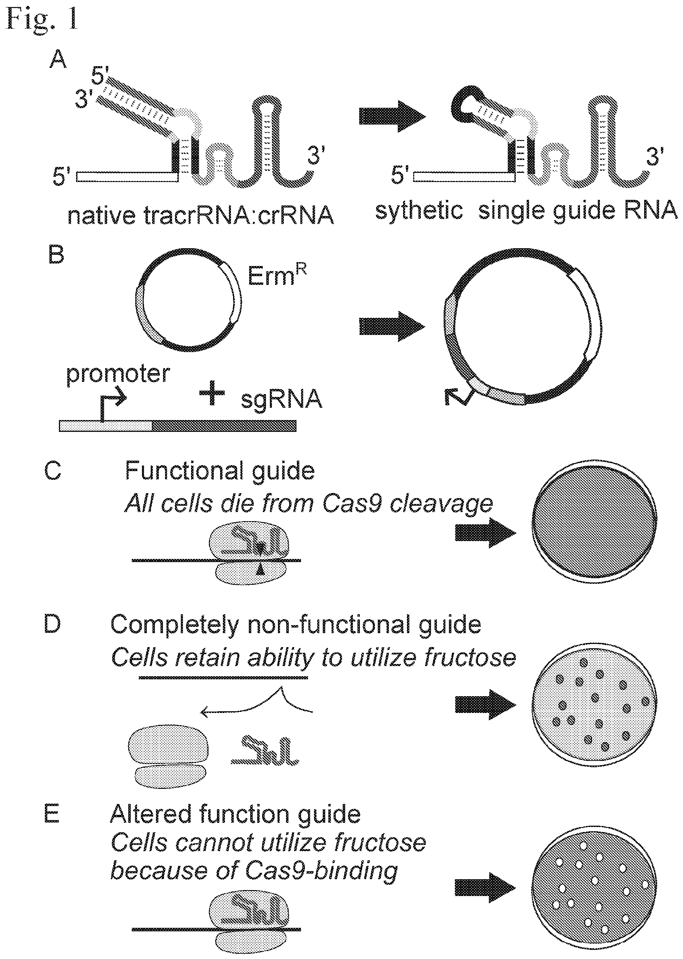

Synthetic single guide RNAs (sgRNA) were designed for *Lactobacillus gasseri* based on the RNA-Seq confirmed boundaries for the tracrRNA and crRNAs of *L. gasseri* (**Fig. 1****, panel A).** A protospacer sequence flanked by the PAM 5'-cTAAC-3' in the FruK was selected as the target for a chromosomal self-targeting assay. The corresponding spacer sequence was designed in the guide RNA. A highly expressed promoter for the *tuf* gene was cloned in front of the sgRNA (**Fig. 1****, panel B**). Using the transformation protocol for *L. gasseri* in a plasmid interference assay, plasmids containing the promoter and single guide were transformed into the cells. Overnight recovered cells were plated on minimal MRS containing 10% fructose, 3ug/ml erythromycin, and bromocresol purple to assess whether the transformants retained the ability to metabolize fructose.

As shown in **Fig. 1****, panel C,** if the Cas9 is able to utilize the sgRNA, the host chromosome will be cleaved, the cells will die, and there should be no growth on any of the plates. However, if Cas9 is able to utilize the guide to bind the target but can no longer cleave the target DNA, the colonies will be white (**Fig. 1****, panel E**). This is shown as the grey portion of the bars in **Fig. 2****, panel B.** Alternatively, if Cas9 is unable to utilize the guide at all, the colonies will be yellow because the cells can utilize fructose, thereby decreasing the pH of the media, and causing a colorometric change (**Fig. 1****, panel D**).

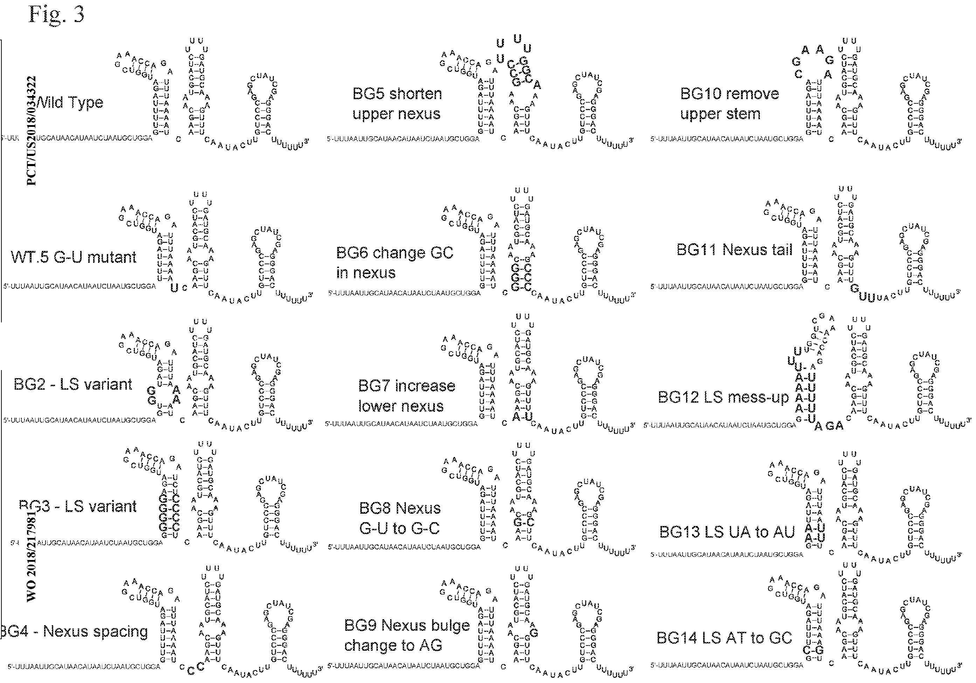

Following the protocol outlined above, single guide RNAs (sgRNAs) based on the *Lactobacillus gasseri* (Lga) Type II crRNA:tracrRNA duplex (**Fig. 2****, panel A**) were designed with mutations made to the stem (e.g., lower stem) and nexus regions (**Fig. 2C** and **Fig. 3**). The sgRNAs were cloned into a plasmid with a high-expression promoter and transformed into Lga to test the ability of the Lga endogenous Cas9 to utilize the sgRNAs. The spacer on the sgRNAs is designed to target the *fruK* (fructose kinase) gene. The recovered cells were plated on a media that differentiates colonies that are able to utilize fructose from those that cannot.

The design of the experiments provides several possible outcomes. (1) When the Cas9 utilizes the sgRNA, the host chromosome is cleaved and the cells die. In this scenario, there should be no growth on any of the plates. (2) When the Cas9 utilizes the guide to bind the target but can no longer cleave the target DNA (resulting from the introduced mutation), the colonies will be white because the cells are unable to ferment lactose, and therefore, there is no decrease of the pH of the media. This is shown as the grey portion of the bars in **Fig. 2****, panel B.** (3) When the Cas9 is unable to utilize the guide at all, the colonies will be yellow because the cells can utilize fructose thereby decreasing the pH of the media and causing a colorometric change. This is shown as the black portion of the bars. In **Fig. 2****, panel B,** the (+) refers to the empty plasmid without a guide RNA and "WT" is Lga transformed with the single guide based on the on the *Lactobacillus gasseri* (Lga) Type II crRNA:tracrRNA duplex without any introduced mutations.

Examples of synthetic modified/mutant guides (e.g., synthetic nucleic acid constructs of the invention) are provided in **Fig. 2****, panel C** and **Fig. 3****.** Example mutant guides that comprise modifications in the nexus hairpin include guide BG4, BG5, BG6, BG7, BG8, BG9, BG11, and BG12 and example mutant guides that comprise modifications in the stem include WT.5, BG2, BG10, BG12, BG13, and BG14.

The results showed that mutant guides WT.5, BG2, BG14, BG4, BG7, BG11, BG10, and BG12 (example nucleic acid constructs of the invention) were utilized by Cas9 and did not allow any transformants to be recovered. Mutant guides BG9, BG6 and BG5 each allowed some Cas9 cleavage, but also provided some binding of the target that prevented the cells from utilizing fructose and mutant guides BG13 and BG8 were more lethal than the WT guide, suggesting improved performance of these guides.

As demonstrated by the mutant guides, increasing the GC content of the nexus appears to allowed the Cas9 polypeptide to utilize the mutant guides, but did not allow the Cas9 to perform complete cleavage (see, e.g., mutant guides BG9, BG6, BG5). Mutations such as these may be useful for repression of transcription. In addition, many of the guides were able to increase the ability of Cas9 to cleave the target DNA and may be useful for improving or increasing Cas9 activity for, for example, killing of cells.

**Table 2. L. gasseri single guide sequences (Fig. 2)**

| | |
|---|---|
| BG WT | TTTAATTGCATAACATAATCTAATGCTGGAgttttagatggtcgaaaccagatttaaa atcaagcaatgcatcttttgatgcaaagtttcaatacttgtcccgagctatcgagggacttttttGGATCCAC A  SEQ ID NO:389 |
| BG 1 LS GU wobble | TTTAATTGCATAACATAATCTAATGCTGGAttttagatggtcgaaaccagatttaaaat caagcaatgcatcttttgatgcaaagtttcaatacttgtcccgagctatcgagggacttttttGGATCCACA SEQ ID NO:390 |
| BG 2 LS V1 | TTTAATTGCATAACATAATCTAATGCTGGAgtGGtagatggtcgaaaccagattta aaatcaagcaatgcatcttttgatgcaaagtttcaatacttgtcccgagctatcgagggacttttttGGATCCA CA SEQ ID NO:391 |
| BG 3 LS V2 | TTTAATTGCATAACATAATCTAATGCTGGAgGGGGagatggtcgaaaccagatc tCCCCtcaagcaatgcatcttttgatgcaaagtttcaatacttgtcccgagctatcgagggacttttttGGATC CACA SEQ ID NO:392 |
| BG4 nexus_spc | TTTAATTGCATAACATAATCTAATGCTGGAgttttagatggtcgaaaccagatttaaa atcCCaagcaatgcatcttttgatgcaaagtttcaatacttgtcccgagctatcgagggacttttttGGATCCA CA SEQ ID NO:393 |
| BG5 nexus_shortu p | TTTAATTGCATAACATAATCTAATGCTGGAgttttagatggtcgaaaccagatttaaa atcaagcaagccttttggcaaagtttcaatacttgtcccgagctatcgagggacttttttGGATCCACA SEQ ID NO:394 |
| BG 6 nexus_lower | TTTAATTGCATAACATAATCTAATGCTGGAgttttagatggtcgaaaccagatttaaa |
| | atcGGgcaatgcatcttttgatgcaaagCCCcaatacttgtcccgagctatcgagggacttttttGGATCC ACA SEQ ID NO:395 |
| BG7 nexus_low_i nc | TTTAATTGCATAACATAATCTAATGCTGGAgttttagatggtcgaaaccagatttaaa atcAaagcaatgcatcttttgatgcaaagtttTcaatacttgtcccgagctatcgagggacttttttGGATCCA CA SEQ ID NO:396 |
| BG8 nexus_GU | TTTAATTGCATAACATAATCTAATGCTGGAgttttagatggtcgaaaccagatttaaa atcaagcaatgcatcttttgatgcaaagCttcaatacttgtcccgagctatcgagggacttttttGGATCCAC A SEQ ID NO:397 |
| BG9 nexusAAtoA G | TTTAATTGCATAACATAATCTAATGCTGGAgttttagatggtcgaaaccagatttaaa atcaagcaatgcatcttttgatgcaaGgtttcaatacttgtcccgagctatcgagggacttttttGGATCCAC A SEQ ID NO:398 |
| BG 10 US-removed | TTTAATTGCATAACATAATCTAATGCTGGAgttttagacgaagatttaaaatcaagca atgcatcttttgatgcaaagtttcaatacttgtcccgagctatcgagggacttttttGGATCCACA SEQ ID NO:399 |
| BG 11 nexus_tail | TTTAATTGCATAACATAATCTAATGCTGGAgttttagatggtcgaaaccagatttaaa atcaagcaatgcatcttttgatgcaaagtttGTTtacttgtcccgagctatcgagggacttttttGGATCCAC A SEQ ID NO:400 |
| BG 12 LS_AUtoUA | TTTAATTGCATAACATAATCTAATGCTGGAgAAAATTTtggtcgaaaccagatT TTTAGAcaagcaatgcatcttttgatgcaaagtttcaatacttgtcccgagctatcgagggacttttttGGAT CCACA SEQ ID NO:401 |
| BG 13 LS_ATtoCG | TTTAATTGCATAACATAATCTAATGCTGGAgCtttagatggtcgaaaccagatttaa aGtcaagcaatgcatcttttgatgcaaagtttcaatacttgtcccgagctatcgagggacttttttGGATCCA CA SEQ ID NO:402 |

**Fig. 4** provides example WT single guides from various bacteria that may be used as templates for modifying single guides as described herein. These guides include that for *Streptococcus pyrogenes, Lactobacillus rhamnosus, Lactobacillus jensenii, Lactobacillus casei*, *Lactobacillus gasseri, Lactobacillus buchneri, Oenococcus kitaharae, Streptococcus thermophilus* CRISPR1, *Lactobacillus animalis* and *Lactobacillus mali.* Similar to the above example showing mutations in the stem and nexus hairpin regions of the *L. gasseri* WT guide, the WT guides for these other organisms may be mutated to provide synthetic mutated guides (e.g., synthetic nucleic acid construct or protein-RNA complex of the invention) having a different functionality from the wild type guide on which the mutated guide was based.

## Claims

1. A synthetic nucleic acid construct, comprising:
(a) a crRNA comprising a 3' region and a 5' region, wherein the 3' region comprises at least about 10 consecutive nucleotides of a CRISPR repeat, and/or a functional fragment of the CRISPR repeat, and the 5' region comprises at least about 20 consecutive nucleotides of a spacer sequence located immediately upstream of the repeat;
(b) a tracrRNA comprising a 5' and a 3' region, wherein at least a portion of the 5' region of the tracrRNA is complementary to the 3' region (i.e., CRISPR repeat) of the crRNA,
wherein, when the 5' region of the tracrRNA that is complementary to the 3' region of the crRNA hybridizes to the 3' region of the crRNA, the synthetic nucleic acid construct forms secondary structures from 5' to 3' of:
(i) a stem, the stem comprising a duplex between the 5' end of the tracrRNA and the repeat of the crRNA, and optionally, the stem further comprising a kink or a bulge;
(ii) a nexus hairpin;
(iii) at least one terminal hairpin;
wherein the construct comprises an insertion, substitution, and/or deletion of about one to about five nucleotides or base pairs in the nexus hairpin as compared to a wild type Type II crRNA:tracrRNA duplex, and wherein the construct comprises a sequence having 100% identity to the synthetic nucleic acid constructs selected from: SEQ ID NO. 408, SEQ ID NO. 407, SEQ ID NO. 405, SEQ ID NO. 409, SEQ ID NO. 406, SEQ ID NO. 404, and SEQ ID NO. 410; and/or
wherein the construct comprises an insertion, substitution and/or deletion of about one to about nine nucleotides and/or base pairs in the stem as compared to a wild type Type II crRNA:tracrRNA duplex, wherein the construct comprises a sequence having 100% identity to the synthetic nucleic acid constructs selected from: SEQ ID NO. 415, SEQ ID NO. 418, SEQ ID NO. 411, SEQ ID NO. 412, SEQ ID NO. 421, SEQ ID NO. 423, and SEQ ID NO: 426; and
optionally, wherein the construct further comprises a linker that when present links the 3'end of the crRNA to the 5' end of the tracrRNA (i.e., linking the 3' end and 5' end of the stem).

2. The synthetic nucleic acid construct of claim 1, wherein the insertion, substitution and/or deletion increases the GC content of the synthetic nucleic acid construct as compared to a wild type Type II crRNA:tracrRNA duplex, and wherein the nucleic acid construct comprises a sequence having 100% identity to the synthetic nucleic acid constructs selected from: SEQ ID NO: 405, SEQ ID NO: 406, SEQ ID NO. 407, and SEQ ID NO: 426.

3. The synthetic nucleic acid construct of claim 1, wherein at least one substitution comprises (a) replacing a complementary base pair with a pair of non-complementary (unmatched) nucleotides (b) replacing a complementary base pair with a different complementary base pair; (c) replacing a pair of non-complementary nucleotides with a different pair of non-complementary nucleotides; or (d) replacing a pair of non-complementary nucleotides with a pair of complementary nucleotides.

4. A protein-RNA complex comprising:
(a) a Cas9 polypeptide; and
(b) the synthetic nucleic acid construct of any one of claims 1 to 3,
optionally wherein the Cas9 has reduced catalytic activity (less or no cleaving/killing) when complexed with the synthetic nucleic acid construct as compared to when the Cas9 is complexed with a wild type crRNA:tracrRNA duplex; or
wherein the Cas9 has increased catalytic activity (increased cleaving/killing) when complexed with the synthetic nucleic acid construct as compared to when the Cas9 is complexed with a wild type crRNA:tracrRNA duplex.

5. An expression cassette comprising:
(a) the synthetic nucleic acid construct of any one of claims 1 to 3 and optionally encoding the Cas9 polypeptide; or
(b) encoding the protein-RNA complex of claim 4.

6. A vector comprising the expression cassette of claim 5.

7. A cell comprising the synthetic nucleic acid construct of any one of claims 1 to 3, the protein-RNA complex of claim 4, the expression cassette of claim 5, or the vector of claim 6, optionally wherein the cell is a plant cell, bacterial cell, fungal cell, animal cell, mammalian cell, insect cell, or archaeon cell.

8. A method of producing a modified Type II crRNA:tracrRNA construct, comprising:
(a) inserting, substituting, and/or deleting about one to about five nucleotides or base pairs in the nexus hairpin of a Type II crRNA:tracrRNA; and/or
(b) inserting, substituting, and/or deleting about one to about nine nucleotides and/or base pairs in a stem of the Type II crRNA:tracrRNA,
wherein the crRNA comprises a 3' region and a 5' region, the 3' region comprising at least about 10 consecutive nucleotides of a CRISPR repeat, and/or a functional fragment of the repeat, and the 5' region comprises at least about 20 consecutive nucleotides of a spacer sequence located immediately upstream of the repeat, and the tracrRNA comprises a 5' and a 3' region, and at least a portion of the 5' region of the tracrRNA is complementary to the 3' region (i.e., CRISPR repeat) of the crRNA,
further wherein, when the 5' region of the tracrRNA that is complementary to the 3' region of the crRNA hybridizes to the 3' region of the crRNA, the Type II crRNA:tracrRNA forms secondary structures from 5' to 3' of:
(i) a stem, the stem comprising a duplex between the 5' end of the tracrRNA and the repeat of the crRNA, and optionally, the stem further comprising a kink or a bulge;
(ii) a nexus hairpin;
(iii) at least one terminal hairpin; and
wherein the nexus hairpin of the Type II crRNA:tracrRNA construct comprises a sequence having 100% identity to the synthetic nucleic acid constructs selected from: SEQ ID NO. 408, SEQ ID NO. 407, SEQ ID NO. 405, SEQ ID NO. 409, SEQ ID NO. 406, SEQ ID NO. 404, and SEQ ID NO. 410; and/or
wherein the stem of the Type II crRNA:tracrRNA construct comprises a sequence having 100% identity to the synthetic nucleic acid constructs selected from: SEQ ID NO. 415, SEQ ID NO. 418, SEQ ID NO. 411, SEQ ID NO. 412, SEQ ID NO. 421, SEQ ID NO. 423, and SEQ ID NO: 426;
and optionally, wherein a linker that when present links the 3'end of the crRNA to the 5' end of the tracrRNA (i.e., linking the 3' end and 5' end of the stem), thereby producing a modified Type II crRNA:tracrRNAs construct.

9. A modified Type II crRNA:tracrRNA construct produced by the method of claim 8.

10. A method of controlling transcription of a target DNA, comprising:
contacting the target DNA with the synthetic nucleic acid construct of any one of claims 1 to 3 in the presence of a Cas9 polypeptide, or with the protein-RNA complex of claim 4, or an expression cassette or vector comprising the same, wherein the synthetic nucleic acid construct and/or the protein-RNA complex binds to the target DNA, thereby controlling the transcription of the target DNA.

11. A method for site-specific cleavage of a target DNA, comprising:
contacting the target DNA with the synthetic nucleic acid construct of any one of claims 1 to 3 in the presence of a Cas9 polypeptide, or with the protein-RNA complex of claim 4, or an expression cassette or vector comprising the same, thereby producing a site specific cleavage of the target DNA in a region defined by complementary binding of the spacer sequence of the crRNA of said synthetic nucleic acid construct to the target DNA.

12. A method of editing a target DNA, comprising:
contacting the target DNA with the synthetic nucleic acid construct of any one of claims 1 to 3 in the presence of a Cas9 polypeptide, or with the protein-RNA complex of claim 4, or an expression cassette or vector comprising the same, wherein the synthetic nucleic acid construct binds to the target DNA and edits the target DNA, thereby editing the target DNA.

13. A method for site-specific nicking of a (+) strand of a double stranded target DNA, comprising:
contacting the double stranded target DNA with the protein-RNA complex of claim 4,
wherein the Cas9 polypeptide comprises a point mutation in an RuvC active site motif, and the Cas9 polypeptide cleaves the (+) strand of the double stranded DNA, thereby producing a site-specific nick in said double stranded target DNA.

14. A method for site-specific nicking of a (-) strand of a double stranded target DNA, comprising:
contacting the double stranded target DNA with a protein-RNA complex of claim 4,
wherein the Cas9 polypeptide comprises a point mutation in an HNH active site motif, and the Cas9 polypeptide cleaves the (-) strand of the double stranded DNA, thereby producing a site-specific nick in said double stranded target DNA.

15. The method of any one of claims 10 to 14, wherein the Cas9 polypeptide is codon optimized for an organism comprising the target DNA, optionally, wherein the organism is a plant, bacterium, fungus, mammal, insect, or archaeon.

## Patentansprüche

1. Synthetisches Nukleinsäurekonstrukt, umfassend:
(a) eine crRNA, die eine 3'-Region und eine 5'-Region umfasst, wobei die 3'-Region mindestens etwa 10 aufeinanderfolgende Nukleotide eines CRISPR-Repeats und/oder ein funktionelles Fragment des CRISPR-Repeats umfasst und die 5'-Region mindestens etwa 20 aufeinanderfolgende Nukleotide einer Spacer-Sequenz umfasst, die sich unmittelbar stromaufwärts des Repeats befindet;
(b) eine tracrRNA, die eine 5'- und eine 3'-Region umfasst, wobei mindestens ein Abschnitt der 5'-Region der tracrRNA komplementär zu der 3'-Region (d. h. CRISPR-Repeat) der crRNA ist,
wobei, wenn die 5'-Region der tracrRNA, die komplementär zu der 3'-Region der crRNA ist, an die 3'-Region der crRNA hybridisiert, das synthetische Nukleinsäurekonstrukt folgende Sekundärstrukturen von 5' zu 3' ausbildet:
(i) einen Stamm, wobei der Stamm einen Duplex zwischen dem 5'-Ende der tracrRNA und dem Repeat der crRNA umfasst, und wobei der Stamm optional ferner einen Knick oder eine Ausbuchtung umfasst;
(ii) eine Nexus-Haarnadel;
(iii) mindestens eine terminale Haarnadel;
wobei das Konstrukt eine Insertion, Substitution und/oder Deletion von etwa einem bis etwa fünf Nukleotiden oder Basenpaaren in der Nexus-Haarnadel im Vergleich zu einem Wildtyp-Typ-II-crRNA:tracrRNA-Duplex umfasst und wobei das Konstrukt eine Sequenz umfasst, die eine Identität von 100 % mit den synthetischen Nukleinsäurekonstrukten aufweist, ausgewählt aus: SEQ ID NO. 408, SEQ ID NO. 407, SEQ ID NO. 405, SEQ ID NO. 409, SEQ ID NO. 406, SEQ ID NO. 404 und SEQ ID NO. 410; und/oder
wobei das Konstrukt eine Insertion, Substitution und/oder Deletion von etwa einem bis etwa neun Nukleotiden und/oder Basenpaaren in dem Stamm im Vergleich zu einem Wildtyp-Typ-II-crRNA:tracrRNA-Duplex umfasst und wobei das Konstrukt eine Sequenz umfasst, die eine Identität von 100 % mit den synthetischen Nukleinsäurekonstrukten aufweist, ausgewählt aus: SEQ ID NO. 415, SEQ ID NO. 418, SEQ ID NO. 411, SEQ ID NO. 412, SEQ ID NO. 421, SEQ ID NO. 423 und SEQ ID NO: 426; und
wobei optional das Konstrukt ferner einen Linker umfasst, der, wenn vorhanden, das 3'-Ende der crRNA mit dem 5'-Ende der tracrRNA verbindet (d. h. das 3'-Ende und das 5'-Ende des Stamms verbindet).

2. Synthetisches Nukleinsäurekonstrukt nach Anspruch 1, wobei die Insertion, Substitution und/oder Deletion den GC-Gehalt des synthetischen Nukleinsäurekonstrukts im Vergleich zu einem Wildtyp-Typ-II-crRNA:tracrRNA-Duplex erhöht und wobei das Nukleinsäurekonstrukt eine Sequenz umfasst, die eine Identität von 100 % mit den synthetischen Nukleinsäurekonstrukten aufweist, ausgewählt aus: SEQ ID NO: 405, SEQ ID NO: 406, SEQ ID NO. 407 und SEQ ID NO: 426.

3. Synthetisches Nukleinsäurekonstrukt nach Anspruch 1, wobei mindestens eine Substitution (a) Ersetzen eines komplementären Basenpaares durch ein Paar nicht komplementärer (nicht übereinstimmender) Nukleotide, (b) Ersetzen eines komplementären Basenpaares durch ein anderes komplementäres Basenpaar; (c) Ersetzen eines Paares nicht komplementärer Nukleotide durch ein anderes Paar nicht komplementärer Nukleotide; oder (d) Ersetzen eines Paares nicht komplementärer Nukleotide durch ein Paar komplementärer Nukleotide umfasst.

4. Protein-RNA-Komplex, umfassend:
(a) ein Cas9-Polypeptid; und
(b) das synthetische Nukleinsäurekonstrukt nach einem der Ansprüche 1 bis 3,
wobei optional das Cas9 eine reduzierte katalytische Aktivität (weniger oder keine Spaltung/Abtötung) aufweist, wenn es mit dem synthetischen Nukleinsäurekonstrukt komplexiert ist, im Vergleich dazu, wenn das Cas9 mit einem Wildtyp-crRNA:tracrRNA-Duplex komplexiert ist; oder
wobei das Cas9 erhöhte katalytische Aktivität (erhöhte Spaltung/Abtötung) aufweist, wenn es mit dem synthetischen Nukleinsäurekonstrukt komplexiert ist, im Vergleich dazu, wenn das Cas9 mit einem Wildtyp-crRNA:tracrRNA-Duplex komplexiert ist.

5. Expressionskassette, umfassend:
(a) das synthetische Nukleinsäurekonstrukt nach einem der Ansprüche 1 bis 3 und optional das Cas9-Polypeptid kodierend; oder
(b) den Protein-RNA-Komplex nach Anspruch 4 kodierend.

6. Vektor, umfassend die Expressionskassette nach Anspruch 5.

7. Zelle, umfassend das synthetische Nukleinsäurekonstrukt nach einem der Ansprüche 1 bis 3, den Protein-RNA-Komplex nach Anspruch 4, die Expressionskassette nach Anspruch 5 oder den Vektor nach Anspruch 6, wobei die Zelle optional eine Pflanzenzelle, Bakterienzelle, Pilzzelle, Tierzelle, Säugetierzelle, Insektenzelle oder Archaeonzelle ist.

8. Verfahren zum Herstellen eines modifizierten Typ-II-crRNA:tracrRNA-Konstrukts, umfassend:
(a) Inserieren, Substituieren und/oder Deletieren von etwa einem bis etwa fünf Nukleotiden oder Basenpaaren in der Nexus-Haarnadel einer Typ-II-crRNA:tracrRNA; und/oder
(b) Inserieren, Substituieren und/oder Deletieren von etwa einem bis etwa neun Nukleotiden und/oder Basenpaaren in einem Stamm der Typ-II-crRNA:tracrRNA,
wobei die crRNA eine 3'-Region und eine 5'-Region umfasst, wobei die 3'-Region mindestens etwa 10 aufeinanderfolgende Nukleotide eines CRISPR-Repeats und/oder ein funktionelles Fragment des Repeats umfasst und die 5'-Region mindestens etwa 20 aufeinanderfolgende Nukleotide einer Spacer-Sequenz umfasst, die sich unmittelbar stromaufwärts des Repeats befindet, und die tracrRNA eine 5'- und eine 3'-Region umfasst und mindestens ein Abschnitt der 5'-Region der tracrRNA komplementär zu der 3'-Region (d. h. CRISPR-Repeat) der crRNA ist,
wobei ferner, wenn die 5'-Region der tracrRNA, die komplementär zu der 3'-Region der crRNA ist, an die 3'-Region der crRNA hybridisiert, die Typ-II-crRNA:tracrRNA folgende Sekundärstrukturen von 5' zu 3' ausbildet:
(i) einen Stamm, wobei der Stamm einen Duplex zwischen dem 5'-Ende der tracrRNA und dem Repeat der crRNA umfasst, und wobei der Stamm optional ferner einen Knick oder eine Ausbuchtung umfasst;
(ii) eine Nexus-Haarnadel;
(iii) mindestens eine terminale Haarnadel; und
wobei die Nexus-Haarnadel des Typ-II-crRNA:tracrRNA-Konstrukts eine Sequenz umfasst, die eine Identität von 100 % mit den synthetischen Nukleinsäurekonstrukten aufweist, ausgewählt aus: SEQ ID NO. 408, SEQ ID NO. 407, SEQ ID NO. 405, SEQ ID NO. 409, SEQ ID NO. 406, SEQ ID NO. 404 und SEQ ID NO. 410; und/oder
wobei der Stamm des Typ-II-crRNA:tracrRNA-Konstrukts eine Sequenz umfasst, die eine Identität von 100 % mit den synthetischen Nukleinsäurekonstrukten aufweist, ausgewählt aus: SEQ ID NO. 415, SEQ ID NO. 418, SEQ ID NO. 411, SEQ ID NO. 412, SEQ ID NO. 421, SEQ ID NO. 423 und SEQ ID NO: 426;
und wobei optional ein Linker, der, wenn vorhanden, das 3'-Ende der crRNA mit dem 5'-Ende der tracrRNA verbindet (d. h. das 3'-Ende und das 5'-Ende des Stamms verbindet), wodurch ein modifiziertes Typ-II-crRNA:tracrRNA-Konstrukt hergestellt wird.

9. Modifiziertes Typ-II-crRNA:tracrRNA-Konstrukt, hergestellt durch das Verfahren nach Anspruch 8.

10. Verfahren zum Steuern einer Transkription einer Ziel-DNA, umfassend:
Inkontaktbringen der Ziel-DNA mit dem synthetischen Nukleinsäurekonstrukt nach einem der Ansprüche 1 bis 3 in der Gegenwart eines Cas9-Polypeptids oder mit dem Protein-RNA-Komplex nach Anspruch 4 oder einer Expressionskassette oder einem Vektor, der diese umfasst, wobei das synthetische Nukleinsäurekonstrukt und/oder der Protein-RNA-Komplex an die Ziel-DNA bindet, wodurch die Transkription der Ziel-DNA gesteuert wird.

11. Verfahren zur ortsspezifischen Spaltung einer Ziel-DNA, umfassend:
Inkontaktbringen der Ziel-DNA mit dem synthetischen Nukleinsäurekonstrukt nach einem der Ansprüche 1 bis 3 in der Gegenwart eines Cas9-Polypeptids oder mit dem Protein-RNA-Komplex nach Anspruch 4 oder einer Expressionskassette oder einem Vektor, der diese umfasst, wodurch eine ortsspezifische Spaltung der Ziel-DNA in einer Region hergestellt wird, die durch komplementäre Bindung der Spacer-Sequenz der crRNA des synthetischen Nukleinsäurekonstrukts an die Ziel-DNA definiert ist.

12. Verfahren zum Editing einer Ziel-DNA, umfassend:
Inkontaktbringen der Ziel-DNA mit dem synthetischen Nukleinsäurekonstrukt nach einem der Ansprüche 1 bis 3 in der Gegenwart eines Cas9-Polypeptids oder mit dem Protein-RNA-Komplex nach Anspruch 4 oder einer Expressionskassette oder einem Vektor, der diese umfasst, wobei das synthetische Nukleinsäurekonstrukt an die Ziel-DNA bindet und die Ziel-DNA editiert, wodurch ein Editing der Ziel-DNA erfolgt.

13. Verfahren zum ortsspezifischen Nicking eines (+)-Strangs einer doppelsträngigen Ziel-DNA, umfassend:
Inkontaktbringen der doppelsträngigen Ziel-DNA mit dem Protein-RNA-Komplex nach Anspruch 4,
wobei das Cas9-Polypeptid eine Punktmutation in einem RuvC-Aktivstellenmotiv umfasst und das Cas9-Polypeptid den (+)-Strang der doppelsträngigen DNA spaltet, wodurch ein ortsspezifischer Nick in der doppelsträngigen Ziel-DNA hergestellt wird.

14. Verfahren zum ortsspezifischen Nicking eines (-)-Strangs einer doppelsträngigen Ziel-DNA, umfassend:
Inkontaktbringen der doppelsträngigen Ziel-DNA mit einem Protein-RNA-Komplex nach Anspruch 4,
wobei das Cas9-Polypeptid eine Punktmutation in einem HNH-Aktivstellenmotiv umfasst und das Cas9-Polypeptid den (-)-Strang der doppelsträngigen DNA spaltet, wodurch ein ortsspezifischer Nick in der doppelsträngigen Ziel-DNA hergestellt wird.

15. Verfahren nach einem der Ansprüche 10 bis 14, wobei das Cas9-Polypeptid ein Codon ist, das für einen Organismus optimiert ist, der die Ziel-DNA umfasst, wobei der Organismus optional eine Pflanze, ein Bakterium, Pilz, Säugetier, Insekt oder Archaeon ist.

## Revendications

1. Construction d'acide nucléique synthétique, comprenant :
(a) un crARN comprenant une région 3' et une région 5', ladite région 3'comprenant au moins environ 10 nucléotides consécutifs d'une répétition de CRISPR, et/ou un fragment fonctionnel de la répétition de CRISPR, et ladite région 5' comprenant au moins environ 20 nucléotides consécutifs d'une séquence d'espacement située immédiatement en amont de la répétition ;
(b) un tracrARN comprenant une région 5' et une région 3', au moins une partie de la région 5'du tracrARN étant complémentaire à la région 3' (c'est-à-dire la répétition CRISPR) du crARN,
lorsque la région 5' du tracrARN qui est complémentaire à la région 3' du crARN s'hybride à la région 3'du crARN, ladite construction d'acide nucléique synthétique formant des structures secondaires de 5' à 3' de :
(i) une tige, la tige comprenant un duplex entre l'extrémité 5' du tracrARN et la répétition du crARN, et éventuellement, la tige comprenant en outre un pli ou un renflement ;
(ii) une épingle à cheveux nexus ;
(iii) au moins une épingle à cheveux terminale ;
ladite construction comprenant une insertion, une substitution et/ou une délétion d'environ un à environ cinq nucléotides ou paires de bases dans l'épingle à cheveux nexus par rapport à un duplex crARN de type II:tracrARN de type sauvage, et ladite construction comprenant une séquence comportant 100 % d'identité avec les constructions d'acide nucléique synthétiques sélectionnées parmi : la SEQ ID n°. 408, la SEQ ID n°. 407, la SEQ ID n°. 405, la SEQ ID n°. 409, la SEQ ID n°. 406, la SEQ ID n°. 404 et la SEQ ID n°. 410 ; et/ou
ladite construction comprenant une insertion, une substitution et/ou une délétion d'environ un à environ neuf nucléotides et/ou paires de bases dans la tige par rapport à un duplex crARN de type Il:tracrARN de type sauvage, ladite construction comprenant une séquence comportant 100 % d'identité avec les constructions d'acide nucléique synthétiques sélectionnées parmi : la SEQ ID n°. 415, la SEQ ID n°. 418, la SEQ ID n°. 411, la SEQ ID n°. 412, la SEQ ID n°. 421, la SEQ ID n°. 423 et la SEQ ID n°. 426 ; et
éventuellement, ladite construction comprenant en outre un lieur qui, lorsqu'il est présent, relie l'extrémité 3' du crARN à l'extrémité 5' du tracrARN (c'est-à-dire, reliant l'extrémité 3'et l'extrémité 5' de la tige).

2. Construction d'acide nucléique synthétique selon la revendication 1, ladite insertion, ladite substitution et/ou ladite délétion augmentant la teneur en GC de la construction d'acide nucléique synthétique par rapport à un duplex crARN de type II:tracrARN de type sauvage, et ladite construction d'acide nucléique comprenant une séquence comportant 100 % d'identité avec les constructions d'acide nucléique synthétiques sélectionnées parmi : la SEQ ID n° : 405, la SEQ ID n°. 406, la SEQ ID n°. 407 et la SEQ ID n°. 426.

3. Construction d'acide nucléique synthétique selon la revendication 1, au moins une substitution comprenant (a) le remplacement d'une paire de bases complémentaires par une paire de nucléotides non complémentaires (non appariés) ; (b) le remplacement d'une paire de bases complémentaires par une paire de bases complémentaires différente ; (c) le remplacement d'une paire de nucléotides non complémentaires par une paire différente de nucléotides non complémentaires ; ou (d) le remplacement d'une paire de nucléotides non complémentaires par une paire de nucléotides complémentaires.

4. Complexe protéine-ARN comprenant :
(a) un polypeptide Cas9 ; et
(b) la construction d'acide nucléique synthétique selon l'une quelconque des revendications 1 à 3,
éventuellement ledit Cas9 comportant une activité catalytique réduite (moins ou pas de clivage/destruction) lorsqu'il est complexé avec la construction d'acide nucléique synthétique par rapport à lorsque le Cas9 est complexé avec un duplex crARN:tracrARN de type sauvage ; ou
ledit Cas9 comportant une activité catalytique accrue (clivage/destruction accrus) lorsqu'il est complexé avec la construction d'acide nucléique synthétique par rapport à lorsque le Cas9 est complexé avec un duplex crARN:tracrARN de type sauvage.

5. Cassette d'expression comprenant :
(a) la construction d'acide nucléique synthétique selon l'une quelconque des revendications 1 à 3 et codant éventuellement le polypeptide Cas9 ; ou
(b) le codage du complexe protéine-ARN selon la revendication 4.

6. Vecteur comprenant la cassette d'expression selon la revendication 5.

7. Cellule comprenant la construction d'acide nucléique synthétique selon l'une quelconque des revendications 1 à 3, le complexe protéine-ARN selon la revendication 4, la cassette d'expression selon la revendication 5 ou le vecteur selon la revendication 6, éventuellement ladite cellule étant une cellule végétale, une cellule bactérienne, une cellule fongique, une cellule animale, une cellule de mammifère, une cellule d'insecte ou une cellule d'archée.

8. Procédé de production d'une construction de crARN de type II:tracrARN modifiée, comprenant :
(a) l'insertion, la substitution et/ou la délétion d'environ un à environ cinq nucléotides ou paires de bases dans l'épingle à cheveux nexus d'un crARN de type II:tracrARN ; et/ou
(b) l'insertion, la substitution et/ou la délétion d'environ un à environ neuf nucléotides et/ou paires de bases dans une tige du crARN de type II:tracrARN,
ledit crARN comprenant une région 3'et une région 5', ladite région 3' comprenant au moins environ 10 nucléotides consécutifs d'une répétition de CRISPR, et/ou un fragment fonctionnel de la répétition, et ladite région 5' comprenant au moins environ 20 nucléotides consécutifs d'une séquence d'espacement située immédiatement en amont de la répétition, et ledit crARN comprenant une région 5' et une région 3', et au moins une partie de la région 5' du crARN étant complémentaire à la région 3' (c'est-à-dire la répétition de CRISPR) du crARN,
en outre, lorsque la région 5' du tracrARN qui est complémentaire à la région 3' du crARN s'hybridant à la région 3'du crARN, ledit crARN de type II:tracrARN formant des structures secondaires de 5' à 3' de :
(i) une tige, la tige comprenant un duplex entre l'extrémité 5'du tracrRNA et la répétition du crARN, et éventuellement, la tige comprenant en outre un pli ou un renflement ;
(ii) une épingle à cheveux nexus ;
(iii) au moins une épingle à cheveux terminale ; et
ladite épingle à cheveux nexus de la construction de crARN de type II:tracrARN comprenant une séquence comportant 100 % d'identité avec les constructions d'acide nucléique synthétiques sélectionnées parmi : la SEQ ID n°. 408, la SEQ ID n°. 407, la SEQ ID n°. 405, la SEQ ID n°. 409, la SEQ ID n°. 406, la SEQ ID n°. 404 et la SEQ ID n°. 410 ; et/ou
ladite tige de la construction de crARN de type II:tracrARN comprenant une séquence comportant 100 % d'identité avec les constructions d'acide nucléique synthétiques sélectionnées parmi : la SEQ ID n°. 415, la SEQ ID n°. 418, la SEQ ID n°. 411, la SEQ ID n°. 412, la SEQ ID n°. 421, la SEQ ID n°. 423 et la SEQ ID n°. 426 ;
et éventuellement, un lieur qui, lorsqu'il est présent, relie l'extrémité 3'du crARN à l'extrémité 5' du tracrARN (c'est-à-dire, reliant l'extrémité 3'et l'extrémité 5' de la tige), produisant ainsi une construction modifiée de crARN de type II:tracrARN.

9. Construction de crARN de type II:tracrARN modifiée produite par le procédé selon la revendication 8.

10. Procédé de contrôle de la transcription d'un ADN cible, comprenant :
la mise en contact de l'ADN cible avec la construction d'acide nucléique synthétique selon l'une quelconque des revendications 1 à 3 en présence d'un polypeptide Cas9, ou avec le complexe protéine-ARN selon la revendication 4, ou une cassette ou un vecteur d'expression comprenant celui-ci , la construction d'acide nucléique synthétique et/ou le complexe protéine-ARN se liant à l'ADN cible, contrôlant ainsi la transcription de l'ADN cible.

11. Procédé de clivage spécifique au site d'un ADN cible, comprenant :
la mise en contact de l'ADN cible avec la construction d'acide nucléique synthétique selon l'une quelconque des revendications 1 à 3 en présence d'un polypeptide Cas9, ou avec le complexe protéine-ARN selon la revendication 4, ou une cassette ou un vecteur d'expression le comprenant, produisant ainsi un clivage spécifique du site de l'ADN cible dans une région définie par une liaison complémentaire de la séquence d'espacement du crARN de ladite construction d'acide nucléique synthétique à l'ADN cible.

12. Procédé d'édition d'un ADN cible, comprenant :
la mise en contact de l'ADN cible avec la construction d'acide nucléique synthétique selon l'une quelconque des revendications 1 à 3 en présence d'un polypeptide Cas9, ou avec le complexe protéine-ARN selon la revendication 4, ou une cassette ou un vecteur d'expression comprenant celui-ci, la construction d'acide nucléique synthétique se liant à l'ADN cible et éditant l'ADN cible, éditant ainsi l'ADN cible.

13. Procédé de coupure spécifique au site d'un brin (+) d'un ADN cible double brin, comprenant :
la mise en contact de l'ADN cible double brin avec le complexe protéine-ARN selon la revendication 4,
ledit polypeptide Cas9 comprenant une mutation ponctuelle dans un motif de site actif RuvC, et ledit polypeptide Cas9 clivant le brin (+) de l'ADN double brin, produisant ainsi une coupure spécifique au site dans ledit ADN cible double brin.

14. Procédé de coupure spécifique au site d'un brin (-) d'un ADN cible double brin, comprenant :
la mise en contact de l'ADN cible double brin avec un complexe protéine-ARN selon la revendication 4,
ledit polypeptide Cas9 comprenant une mutation ponctuelle dans un motif de site actif HNH, et ledit polypeptide Cas9 clivant le brin (-) de l'ADN double brin, produisant ainsi une coupure spécifique au site dans ledit ADN cible double brin.

15. Procédé selon l'une quelconque des revendications 10 à 14, ledit polypeptide Cas9 étant un codon optimisé pour un organisme comprenant l'ADN cible, éventuellement, ledit organisme étant une plante, une bactérie, un champignon, un mammifère, un insecte ou une archée.
